# EUROPEAN PATENT SPECIFICATION

(11) **EP 4 480 394 B1**
(45) Date of publication and mention of the grant of the patent: **25.02.2026**
(21) Application number: 24182458.0
(22) Date of filing: 17.06.2024
(51) Int. Cl.: A61B 5/00, A61B 7/04, A61B 5/11

(54) **SYSTEM FOR MONITORING HEART FUNCTION BASED ON ELECTROMECHANICAL ACTIVATION TIME**
SYSTEM ZUR ÜBERWACHUNG DER HERZFUNKTION AUF DER BASIS DER ELEKTROMECHANISCHEN AKTIVIERUNGSZEIT
SYSTÈME DE SURVEILLANCE DE FONCTION CARDIAQUE SUR LA BASE D'UN TEMPS D'ACTIVATION ÉLECTROMÉCANIQUE

(30) Priority: 22.06.2023 US 202363509718 P; 14.06.2024 US 202418743533
(43) Date of publication of application: 25.12.2024
(73) Proprietor: Pacesetter, Inc., Sylmar, CA 91342 (US)
(72) Inventor: POLITIS, Nikolaos, Berlin (DE); GILL, Jong, Valencia (US)
(74) Representative: Barker Brettell Sweden AB

(56) References cited:
- WO-A1-2020/033683
- US-A1- 2008 273 709
- US-A1- 2012 296 228
- US-A1- 2013 060 150
- US-A1- 2013 237 863
- US-A1- 2022 280 123

## Description

### FIELD OF THE INVENTION

Embodiments of the present disclosure generally relate to methods and systems for monitoring heart function based on heart sounds.

### BACKGROUND OF THE INVENTION

Implantable medical devices (IMDs) are offered today for a wide variety of applications to monitor and treat various physiologic conditions. More recently, an interest has developed in utilizing heart sounds as a cardiac biomarker, such as in connection with providing clinically useful information related to ventricular contraction in various valve related diseases.

Miniaturized accelerometers have been proposed, that utilize microelectromechanical system (MEMS) technology, to detect heart sounds while the accelerometers are implanted within an IMD. Conventional heart sound monitoring techniques generally monitor aspects such as the heart sound duration, heart sound amplitude, intervals between heart sound peaks, intervals between an R-wave peak and a heart sound peak and the like.

One use of the MEMS technology is the prediction of heart failure (HF) using heart sounds. Heart sounds are an effect of heart valve closures caused by the contraction of the heart and occur after QRS complex for S1 and after T wave for S2. S1 heart sounds are shown to correlate with left ventricular contraction and are associated with the vibrational sound made by mitral and tricuspid valves. S2 heart sounds are typically associated with the closure of aortic valve, and the presence of S3 component can be an early sign of HF. Generally, heart sounds can be used to track proper or improper functioning of a patient's heart, and thus can be used to support HF diagnostics. Still, obtaining accurate heart sound data remains difficult.

A need remains for improvements in monitoring heart function based on heart sounds and utilizing the heart sound for diagnostic purposes.

US 2013/0060150 discloses a medical device system and associated method discriminating conditions. The system includes a plurality of sensors, a sensing module coupled to the plurality of sensors and configured to acquire signals from the plurality of sensors, and a processor coupled to the sensing module and configured to determine a change in a respiratory related sounds in response to a signal from a sensor of the plurality of sensors, select one or more sensors of the plurality of sensors associated with the determined change, determine a respiratory signature in response to signals from the selected one or more sensors, and determine the respiratory condition in response to the determined respiratory signature.

US 2012/0296228 discloses an IMD receiving both heart sound and electrogram signals. A processor within the implantable medical device extracts physiologically relevant information from both the heart sound signal and the electrogram signal. Based on the extracted physiologically relevant information a set of pacing parameters is evaluated. The values of the pacing parameters may be changed by the IMD in response to the physiologically relevant information extracted from the heart sound signal and the electrogram signal.

WO 2020/033683 discloses a computer implemented method and system for detecting arrhythmias in cardiac activity. The method is under control of one or more processors configured with specific executable instructions. The method obtains cardiac activity (CA) signals at the electrodes of an IMD in connection multiple cardiac beats and with different IMD orientations relative to gravitational force. The method obtains acceleration signatures at a sensor of the IMD that are indicative of heart sounds generated during the cardiac beats. The method obtains device location information at the IMD, with respect to the gravitational force during the cardiac beats. The method groups the acceleration signatures associated with the first and second set of cardiac beats into the corresponding one of first and second posture bins based on the device location information. The method identifies a difference between the acceleration signals in the first posture bin in connection with treating a heart condition.

US 2008/273709 A1 discloses a tunable auscultation system including a heart sound acquirer for sensing heart sounds from at least one chest location of the patient. An initial conditioner then conditions the heart sounds through preamplification and anti-aliasing. The heart sounds are transduced into electrical signals by a signal processor. The electric heart signals are then tuned by an analysis tool. The analysis tool includes an interaction tuner, a processing tuner and an output tuner. The interaction tuner includes a preset tuning selector and a dynamic range tuning selector. The processing tuner includes a band pass filter and an algorithmic extraction engine which applies extraction algorithms to the electric heart signals, segments them and extracts signals of interest. Signals of interest may be correlated to specific pathologies. The output tuner includes a signal strength indicator, a diagnosis indicator, an overlapping cardiac cycle display and a display configuration engine. A display module provides output.

### SUMMARY

The present invention is defined in the independent claim. Further embodiments of the invention are defined in the dependent claims. Aspects, embodiments and examples disclosed herein which do not fall within the scope of the appended claims do not form part of the invention and are merely provided for illustrative purposes.

In accordance with embodiments herein, an implantable medical device (IMD) is provided. The IMD includes a HS sensor configured to sense heart sound (HS) signals along an axis over a first period of time and a filtering assembly configured to filter the HS signals utilizing first and second bandwidths to output first and second bandwidth HS components. The IMD also includes a memory to store specific executable instructions and one or more processors. When executing the specific executable instructions, the one or more processors can be configured to identify a first characteristic of interest (COI) of a heartbeat from the first bandwidth HS component and identify a second COI of the heartbeat from the second bandwidth HS component wherein the second COI is a same COI of a heartbeat as the first COI. The one or more processors are configured to select one of the first and second bandwidths based on a comparison of the first and second COI, obtain additional HS signals during a second period of time and utilize the one of the first and second bandwidths selected to filter the additional HS signals.

Optionally, the first period of time can include first and second intervals, the first bandwidth HS component sensed during the first interval, the second bandwidth HS component sensed during the second interval.

Optionally, the first bandwidth can have a different frequency range than the second bandwidth.

Optionally, the additional HS signals may not be filtered utilizing the one of the first and second bandwidths that is not selected.

Optionally, the one or more processors can be further configured to determine a first electromechanical activation time (EMAT) based on the first COI of the heartbeat.

Optionally, the axis can be a first axis and the HS sensor includes a second axis and a third axis, and the one or more processors are further configured to identify a third COI of the heartbeat from a third bandwidth component related to the second axis, identify a fourth COI of the heartbeat from a fourth bandwidth component related to the second axis, select one of the first, second, third, or fourth bandwidth components based on a comparison of the first, second, third, and fourth COI and utilize the one of the first, second, third or fourth bandwidth components selected to filter the additional HS signals.

Optionally, the one or more processors can be further configured to identify a fifth COI of the heartbeat from a fifth bandwidth component, identify a sixth COI of the heartbeat from a sixth bandwidth component, select one of the first, second, third, fourth, fifth, or sixth bandwidth components based on a comparison of the first, second, third, fourth, fifth, or sixth COI and utilize the one of the first, second, third, fourth, fifth, or sixth bandwidth components selected to filter the additional HS signals.

Optionally, the one or more processors are further configured to treat a physiologic condition based on the additional HS signals.

Optionally, the first COI can be selected from the group consisting of S1 amplitude, S2 amplitude, S3 amplitude, S1 to S2 interval, S2 to S3 interval, and S3 to S1 interval.

Optionally, the one or more processors can be further configured to operate the IMD in a standby mode wherein no HS signals are sensed, operate the IMD in a single-axis mode wherein HS signals are only obtained from the first axis and operate in a three-axis mode wherein HS signals are sequentially obtained from the first axis and then from the second axis.

Optionally, the one or more processors can be further configured to obtain HS data from the additional HS signals and communicate the HS data to a first external device.

Optionally, to obtain the HS data from the additional HS signals, the one or more processors may be further configured to determine that a patient is sleeping based on one-dimensional activity or trended posture measurements and record the additional HS signals for a determined period in response to determining that the patient is sleeping.

Optionally, the one or more processors can be further configured to monitor the patient during the determined period, determine that the patient is awake, and stop recording the additional HS signals in response to determining that the patient is awake.

Optionally, to determine the patient is sleeping, the one or more processors can be further configured to analyze the additional HS signals.

Optionally, the first external device is a proximate external device, such as can be proximate to a patient.

Optionally, the one or more processors can be further configured to communicate the HS data to a second external device.

Optionally, the second external device may be a remote external device.

Optionally, to select one of the first and second bandwidths includes determining a first ensemble average HS signal related to the first bandwidth HS component and determining a second ensemble average HS signal related to the second bandwidth HS and comparing the first ensemble average HS signal and the second ensemble average HS signal.

Optionally, the one or more processors can be further configured to determine a first electromechanical activation time (EMAT) based on the first COI of the heartbeat, determine a second EMAT based on the second COI of the heartbeat, select one of the first EMAT or the second EMAT to provide a selected EMAT based on a shared characteristic of the first EMAT and second EMAT and obtain the additional HS signals during the second period of time based on the selected EMAT.

Optionally, the one or more processors can be further configured to determine a first electromechanical activation time (EMAT) based on the first bandwidth HS component, determine a second EMAT based the second bandwidth HS component, determine a third EMAT based on a third bandwidth HS component, select one of the first EMAT, the second EMAT, or the third EMAT to provide a selected EMAT based on a shared characteristic of the first EMAT, second EMAT, and third EMAT and obtain the additional HS signals based on the selected EMAT.

Optionally, the one or more processors can be further configured to determine a first electromechanical activation time (EMAT) based on the first bandwidth HS component and the first COI of the heartbeat, determine at least one of 1) a second EMAT based on the second bandwidth HS component and the second COI of the heart beat or 2) a third EMAT based on a third bandwidth HS component and a third COI of the heartbeat, select one of the first EMAT, the second EMAT, or the third EMAT to provide a selected EMAT based on a shared characteristic of the first EMAT, second EMAT, and third EMAT and obtain the additional HS signals based on the selected EMAT.

Optionally, to select one of the first EMAT, the second EMAT, or the third EMAT to provide a selected EMAT, the one or more processors can be further configured to determine a migration of the IMD.

Optionally, the one or more processors can be further configured, when in a therapy mode, to collect and analyze HS signals to identify an HS of interest on a beat-by-beat basis.

Optionally, the HS sensor can be a three-axis accelerometer.

Optionally, the one or more processors can be further configured to obtain HS data from the additional HS signals and communicate the HS data to a first external device.

Optionally, to obtain the HS data from the additional HS signals, the one or more processors can be further configured to determine that a patient is sleeping based on one-dimensional activity or trended posture measurements and record the additional HS signals for a determined period in response to determining that the patient is sleeping.

In accordance with embodiments herein, an IMD is provided that includes a HS sensor configured to sense heart sound (HS) signals within first and second bandwidths and along a first axis of the HS sensor over the first period of time, a memory to store specific executable instructions, and one or more processors. When executing the specific executable instructions, the one or more processors are configured to obtain, during a first period, a first characteristic of interest (COI) of each heartbeat of a series of heartbeats over a first interval. The one or more processors are also configured to analyze the first COI of each heartbeat of the series of heartbeats to one another to determine a first variance and determine whether to utilize a first bandwidth HS component to obtain the first COI of additional heartbeats during a second period based on the first variance.

Optionally, the first variance can be one of a standard deviation or a covariance.

Optionally, determining whether to utilize the first bandwidth HS component can include comparing the first variance to a threshold related to the first variance, and utilizing the first bandwidth HS component to obtain the first COI of the additional heartbeats when the threshold is met.

Optionally, the one or more processors can be further configured to obtain, utilizing a second bandwidth HS component during the first period, a second COI of each heartbeat of a series of heartbeats over a second interval in response to determining not to utilize the first bandwidth HS component during the second period, analyze the second COI of each heartbeat of the series of heartbeats to one another to determine a second variance, and determine whether to utilize the second bandwidth HS component to obtain the second COI of the additional heartbeats during the second period based on the second variance.

In accordance with embodiments herein, a computer implemented method for monitoring heart function based on heart sounds (HS) in an implantable medical device IMD is provided. Said method does not fall within the claimed subject-matter and does not form part of the invention. The method can include obtaining electrical cardiac activity (CA) signals, sensed at implantable electrodes provided on the IMD, over a first period of time and a second period of time, obtaining HS signals, sensed by an implantable HS sensor, over the first period of time, identifying a first characteristic of interest (COI) of a heartbeat from the HS signals obtained over the first period of time and related to a first bandwidth HS component, identifying a second COI of the heartbeat obtained from the HS signals over the first period of time and related to a second bandwidth HS component, and determining whether to obtain additional HS signals during the second period of time utilizing the first bandwidth HS component or the second bandwidth HS component based on the first COI and the second COI.

Optionally, determining whether to obtain the HS signals utilizing the first bandwidth HS component, or from the second bandwidth HS component can include comparing the first COI to the second COI.

Optionally, the first period of time can include a first interval and a second interval that can be different than the first interval, and the first COI can be identified based on the HS signals obtained during the first interval and the second COI can be based on the HS signals obtained during the second interval.

Optionally, the method also includes identifying a third COI of the heartbeat from the HS signals obtained over the first period of time and related to a third bandwidth HS component, identifying a fourth COI of the heartbeat obtained from the HS signals over the first period of time and related to a fourth bandwidth HS component, and determining whether to obtain the HS signals from the first bandwidth HS component, the second bandwidth HS component, the third bandwidth HS component, or the fourth bandwidth HS component based on the first COI, the second COI, the third COI, and the fourth COI, respectfully.

Optionally, the method also includes identifying a fifth COI of the heartbeat from the HS signals obtained over the first period of time and related to a fifth bandwidth HS component, identifying a sixth COI of the heartbeat obtained from the HS signals over the first period of time and related to a sixth bandwidth HS component, and determining whether to obtain the HS signals utilizing the first, second, third, fourth, fifth, or sixth bandwidth HS component based on the first, second, third, fourth, fifth, and sixth COls of the heartbeat.

Optionally, the first COI can be selected from the group consisting of S1 amplitude, S2 amplitude, S3 amplitude, S1 to S2 interval, S2 to S3 interval, and S3 to S1 interval.

Optionally, the method can also include operating the IMD in a standby mode wherein no HS signals are sensed, operating the IMD in a single-axis mode wherein HS signals are only obtained from the first axis, and operating in a three-axis mode wherein HS signals are sequentially obtained from the first axis and then from the second axis.

Optionally, the method also can include obtaining HS data from the additional HS signals and communicating the HS data to a first external device.

Optionally, obtaining the HS data from the additional HS signals can include determining that a patient is sleeping based on one-dimensional activity or trended posture measurements, and recording the additional HS signals for a determined period in response to determining that the patient is sleeping.

Optionally, the method can also include monitoring the patient during the determined period, determining that the patient is awake, and stopping recording of the additional HS signals in response to determining that the patient is awake.

Optionally, determining whether the patient is sleeping includes analyzing the additional HS signals.

Optionally, determining whether to obtain the HS signals during the second period of time utilizing the first bandwidth HS component or the second bandwidth HS component can include determining during the first period of time a first ensemble average HS signal related to the first bandwidth HS component, and determining a second ensemble average HS signal related to the second bandwidth HS component.

Optionally, the one or more processors can be further configured to monitor the patient during the determined period, determine that the patient is awake, and stop recording the additional HS signals in response to determining that the patient is awake.

Optionally, to determine whether the patient is sleeping, the one or more processors can be further configured to analyze the additional HS signals.

Optionally, the first external device can be a proximate external device, such as can be proximate to a patient.

Optionally, the first external device is a clinician device.

Optionally, the one or more processors can be further configured to communicate the HS data to a second external device.

Optionally, the second external device can be a remote external device.

In accordance with embodiments herein, a computer implemented method for monitoring heart function based on heart sounds (HS) in an IMD is provided. Said method does not fall within the claimed subject-matter and does not form part of the invention. The method can include obtaining electrical cardiac activity (CA) signals, sensed at implantable electrodes provided on the IMD, over a period of time, obtaining HS signals, sensed by an implantable HS sensor, over the period of time, identifying a characteristic of interest (COI) of a heartbeat from the HS signals, determining a first electromechanical activation time (EMAT) based on a first bandwidth HS component, determining at least one of 1) a second EMAT based on a second bandwidth HS component or 2) a third EMAT based on a third bandwidth HS component, selecting one of the first EMAT, the second EMAT, or the third EMAT to provide a selected EMAT based on a shared characteristic of the first EMAT, second EMAT, and third EMAT, and obtaining additional HS signals based on the selected EMAT.

Optionally, the method also includes obtaining HS data from the additional HS signals and communicating the HS data to a first external device.

Optionally, obtaining the HS data from the additional HS signals can include determining that a patient is sleeping based on one-dimensional activity or trended posture measurements, and recording the additional HS signals for a determined period in response to determining that the patient is sleeping.

Optionally, the method can include monitoring the patient during the determined period, determining that the patient is awake, and stopping the recording of the additional HS signals in response to determining that the patient is awake.

### BRIEF DESCRIPTION OF THE DRAWINGS

Figure 1 illustrates a schematic block diagram of an IMD intended for subcutaneous implantation at a site near the heart.
Figure 2 illustrates an example block diagram of the IMD of Figure 1 formed in accordance with embodiments herein.
Figure 3A illustrates graphs of raw HS data overlaid on top of a surface ECG signal in accordance with embodiments herein.
Figure 3B illustrates graphs of an EMAT in accordance with embodiments herein.
Figure 4 illustrates a schematic diagram of a HS sensor implemented as an accelerometer in accordance with embodiments herein.
Figure 5 provides a sectional view of a patient's heart and shows a leadless implantable medical in accordance with embodiments herein.
Figure 6 illustrates a side view of the IMD of Figure 5 in accordance with embodiments herein.
Figure 7 illustrates a graph of a HS signal in accordance with embodiments herein.
Figure 8 illustrates a graph of a HS signal in accordance with embodiments herein.
Figure 9 illustrates a schematic block flow diagram of a method for determining an EMAT utilizing HS data in accordance with embodiments herein.
Figure 10 illustrates a schematic diagram of a system, in accordance with embodiments herein.
Figure 11 illustrates a schematic block diagram of a system for verifying an update sent by a clinician, in accordance with embodiments herein.
Figure 12 illustrates a schematic block flow diagram of a system operated, in accordance with embodiments herein.
Figure 13 illustrates a schematic block diagram of an electronic device, in accordance with embodiments herein.
Figure 14 illustrates a schematic block flow diagram of a system operated, in accordance with embodiments herein.
Figure 15 illustrates a schematic block flow diagram of a process for obtaining HS data from a 3-D accelerometer, in accordance with embodiments herein.
Figure 16A illustrates a schematic block flow diagram of a process for obtaining HS data from a 3-D accelerometer, in accordance with embodiments herein.
Figure 16B illustrates a schematic block flow diagram of a process for obtaining HS data from a 3-D accelerometer, in accordance with embodiments herein.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein, which do not form part of the claimed invention, may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all such operations may be performed by the processor(s) of another device described herein. For example, an IMD includes IMD memory and one or more IMD processors, while each external device/system (ED) (e.g., local, remote, or anywhere within the healthcare system) includes ED memory and one or more ED processors.

### Terms

The terms "posture" and "patient posture" refer to postural states and/or activity levels of a patient including supine, laying on a right side, laying on a left side, sitting, standing, isometric arm exercises (e.g., pushing, pulling, and the like), ballottement, chest thump, device pressure (e.g., top, mid, and base), arm flap, handshake, and the like.

The term "activity level" refers to intensity and/or types of activity currently experienced by a patient at a point in time, including stationary state, rest state, exercise state, walking state, and the like.

The terms "cardiac activity signal", "cardiac activity signals", "CA signal" and "CA signals" (collectively "CA signals") are used interchangeably throughout to refer to an analog or digital electrical signal recorded by two or more electrodes positioned subcutaneous or cutaneous, where the electrical signals are indicative of cardiac electrical activity. The cardiac activity may be normal/healthy or abnormal/arrhythmic. Non-limiting examples of CA signals include ECG signals collected by cutaneous electrodes, and EGM signals collected by subcutaneous electrodes and/or by electrodes positioned within or proximate to the heart wall and/or chambers of the heart.

The term "electromechanical activation time" or "EMAT" are used interchangeably throughout to refer to an interval from the beginning of electrical activation of the left ventricle (LV) to the onset of the first heart sound created because of the closure of the mitral valve. The beginning of the electrical activation of the LC can be considered the onset of the ECG Q wave. So, when an S1 heart sound is combined with the ECG the EMAT can be measured.

The terms "health care system" and "digital health care system" are used interchangeably throughout to reference to a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. Pat. Pub. No. 2021/0020294 entitled METHODS DEVICE AND SYSTEMS FOR HOLISTIC INTEGRATED HEALTHCARE PATIENT MANAGEMENT. Other patents that describe example monitoring systems include U.S. Pat. No. 6,572,557; entitled SYSTEM AND METHOD FOR MONITORING PROGRESSION OF CARDIAC DISEASE STATE USING PHYSIOLOGIC SENSORS; U.S. Pat. No. 6,480,733 entitled METHOD FORMONITORING HEART FAILURE; U.S. Pat. No. 7,272,443 entitled SYSTEM AND METHOD FOR PREDICTING A HEART CONDITION BASED ON IMPEDANCE VALUES USING AN IMPLANTABLE MEDICAL DEVICE; U.S. Pat. No. 7,308,309 entitled DIAGNOSING CARDIAC HEALTH UTILIZING PARAMETER TREND ANALYSIS; and U.S. Pat. No. 6,645,153 entitled SYSTEM AND METHOD FOR EVALUATING RISK OF MORTALITY DUE TO CONGESTIVE HEART FAILURE USING PHYSIOLOGIC SENSORS.

The term "obtains" and "obtaining", as used in connection with data, signals, information and the like, include at least one of i) accessing memory of an external device or remote server where the data, signals, information, etc. are stored, ii) receiving the data, signals, information, etc. over a wireless communications link between the IMD and a local external device, and/or iii) receiving the data, signals, information, etc. at a remote server over a network connection. The obtaining operation, when from the perspective of an IMD, may include sensing new signals in real time, and/or accessing memory to read stored data, signals, information, etc. from memory within the IMD. The obtaining operation, when from the perspective of a local external device, includes receiving the data, signals, information, etc. at a transceiver of the local external device where the data, signals, information, etc. are transmitted from an IMD and/or a remote server. The obtaining operation may be from the perspective of a remote server, such as when receiving the data, signals, information, etc. at a network interface from a local external device and/or directly from an IMD. The remote server may also obtain the data, signals, information, etc. from local memory and/or from other memory, such as within a cloud storage environment and/or from the memory of a workstation or clinician external programmer.

The terms "artificial intelligence", "machine learning" and "self-learning" are used interchangeably throughout and shall mean an artificial intelligence algorithm that learns from various automatic or manual inputs, such as features of interest, prior device classified arrhythmias, observations and/or data. The machine learning algorithm is adjusted over multiple iterations based on the features of interest, posture, heart sound signals, S1 COM, S2 COM, EMAT, SI, CA signals, characteristics of interest of the CA signals, prior device classified arrhythmias, observations and/or data. For example, the machine learning algorithm is adjusted by supervised learning, unsupervised learning, and/or reinforcement learning. Non-limiting examples of machine learning algorithms are a convolutional neural network, gradient boosting random forest, decision tree, K-means, deep learning, artificial neural network, and/or the like.

The term "subcutaneous" shall mean below the skin, but not intravenous. For example, a subcutaneous electrode/lead does not include an electrode/lead located in a chamber of the heart, in a vein on the heart, or in the lateral or posterior branches of the coronary sinus.

The terms "RA", "LA", "RV", and "LV" shall mean the right atrium, left atrium, right ventricle, and left ventricle, respectively.

The term "leadless" generally refers to an absence of electrically conductive leads that traverse vessels or other anatomy outside of the intra-cardiac space, while "intra-cardiac" means generally, entirely within the heart and associated vessels, such as the superior vena cava (SVC), inferior vena cava (IVC), coronary sinus (CS), coronary veins (CV), pulmonary arteries, and the like.

The term "COI" refers to a characteristic of interest within a signal. Non-limiting examples of COI from a CA signal with a PQRST complex, include an R-wave, P-wave, T-wave, and isoelectric segments. Non-limiting examples of COI from CA signals collected at an individual electrode(s) include a sensed event (e.g., an intrinsic event or evoked response). The COI may correspond to a peak of an individual sensed event, R-wave, an average or median P, R or T-wave peak and the like. In another example, the COI may include COIs of HS signals including S1 amplitudes, S2 amplitudes, S3 amplitudes, S4 amplitudes, S1 to S2 intervals, S2 to S3 intervals, S3 to S4 intervals, S4 to S1 intervals, or the like.

### Overview

In accordance with new and unique aspects herein, methods and devices are described that incorporate an accelerometer (AXL) into an implantable medical device, such as an implantable cardiac monitor (ICM), to monitor and utilize heart sounds (HS) for diagnostic and treatment purposes. Said methods do not fall within the claimed subject-matter and do not form part of the claimed invention. Heart sounds are an important cardiac biomarker that provides clinically useful information related to ventricular contraction and various valve related diseases. Miniaturized AXLs using micro-electro-mechanical-system (MEMS) technology made HS detection possible from implantable medical devices. Implantable cardiac monitor (ICM) devices can incorporate a three-dimensional (3-D) AXL capable of simultaneously recording HS together with electrocardiogram (ECG). The S1 HS is a surrogate for the beginning of systole. When combined with ECG, it can be used to measure electromechanical activation time (EMAT). The same AXL can be utilized to detect movement of the patient during day-to-day activities, the posture based on the position and/or orientation of the ICM and the migration of the device. This disclosure describes different methods and system to detect HS signal from different axis of a 3D AXL and calculate EMAT.

In one example embodiment, an HS data collection feature is the collection of HS data from the 3D AXL by an IMD such as an ICM and the retrieval and storage of the data by an external instrument, such as a remote device. The 3D AXL supports the following modes: standby, single axis (1D activity), normal (3D activity/posture), and HS. Each mode is exclusive (only one mode can occur at a time), hence HS data collection cannot occur in parallel with 3D activity and posture. In HS mode, the 3D AXL can detect HS in three axes (X, Y, Z) and in two HS bandpass filter settings: wideband (WB), (e.g., with a frequency range of 7.5 - 100 Hz), and narrowband (NB), (e.g., with a frequency range of 15 - 100 Hz), for a total of six bandwidth HS components. The HS sensor can be configured to output one bandwidth HS component at a time; hence, HS data can be collected sequentially for each of the six bandwidth HS components rather than collecting data for all six bandwidth HS components at once.

In another example embodiment an IMD can incorporate a 3-D AXL capable of detecting HS in 3 orthogonal (X, Y and Z) axes. However, it is not always easy to rely on only one axis to detect the consistent timing of EMAT. For example, depending on the shape and nature of the device pocket, it is quite possible that there exists a patient specific optimal axis that is unique to the patient. In one example, patient posture, or even possible migration of the device, can influence the timing and quality of HS data and subsequent EMAT measurements. Presented are methods of incorporating all 3 axis and 2 bandwidths and implementing a HS analyzer to determine EMAT based on metrics determined by the positional data from the AXL.

### Implantable Medical Device

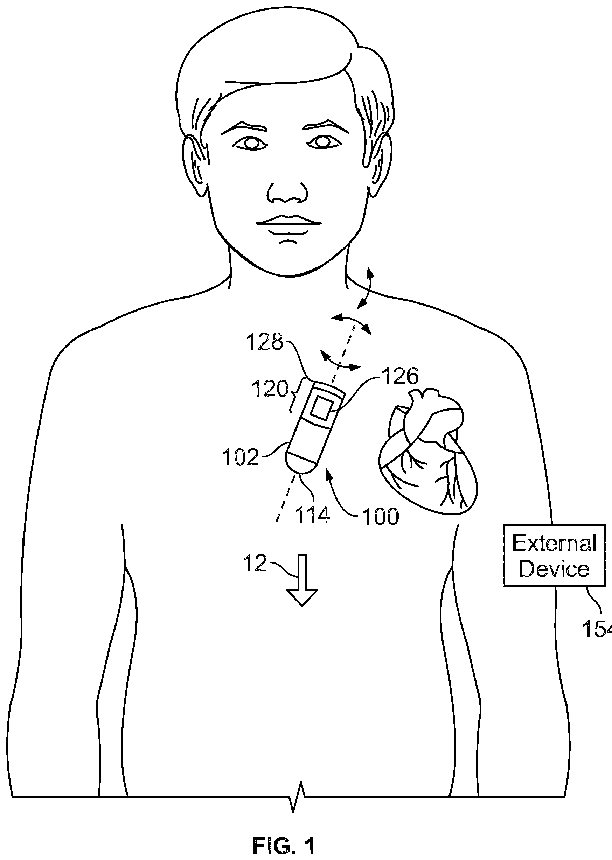

Figure 1 illustrates an IMD 100 intended for subcutaneous implantation at a site near the heart. In one example, the IMD and related sensors, devices, systems, etc. as described herein is at least one of the IMDs, sensors, devices, systems, etc. illustrated and described in U.S. Pub. No. 2022/0361837, entitled METHOD AND SYSTEM FOR MONITORING HEART FUNCTION BASED ON HEART SOUND CENTER OF MASS. In another example, the IMD and related sensors, devices, systems, etc. as described herein is at least one of the IMDs, sensors, devices, systems, etc. illustrated and described in U.S. Pub. No. 2022/0361818, entitled METHOD AND SYSTEM FOR MONITORING HEART FUNCTION BASED ON HEART SOUND CENTER OF MASS.

The IMD 100 includes a pair of spaced-apart sense electrodes 114, 126 positioned with respect to a housing 102. The sense electrodes 114, 126 provide for detection of far field electrogram signals. Numerous configurations of electrode arrangements are possible. For example, the electrode 114 may be located on a distal end of the IMD 100, while the electrode 126 is located on a proximal side of the IMD 100. Additionally or alternatively, electrodes 126 may be located on opposite sides of the IMD 100, opposite ends or elsewhere. The distal electrode 114 may be formed as part of the housing 102, for example, by coating all but a portion of the housing with a nonconductive material such that the uncoated portion forms the electrode 114. In this case, the electrode 126 may be electrically isolated from the housing 102 electrode by placing it on a component separate from the housing 102, such as the header 120. Optionally, the header 120 may be formed as an integral portion of the housing 102. The header 120 includes an antenna 128 and the electrode 126. The antenna 128 is configured to wirelessly communicate with an external device 154 in accordance with one or more predetermined wireless protocols (e.g., Bluetooth, Bluetooth low energy, Wi-Fi, etc.). In one example, the external device 154 is a proximate external device located proximate to or adjacent to a patient. An external device located in the same room as the patient is a proximate external device. Alternatively, the external device can be a remote external device where the external device is located a long distance from the such as at least one-hundred meters, in a different room, in a different building, at least 8.05 km (five miles) away, at least 16.09 km (ten miles) away, at least 160.93 km (one-hundred miles) away, etc.

The housing 102 includes various other components such as: sense electronics for receiving signals from the electrodes, a microprocessor for analyzing the far field CA signals, including assessing the presence of R-waves in cardiac beats occurring while the IMD is in different IMD locations relative to gravitational force, a loop memory for temporary storage of CA data, a device memory for long-term storage of CA data, sensors for detecting patient activity, including an AXL for detecting acceleration signatures indicative of heart sound, and a battery for powering components.

In at least some embodiments, the IMD 100 is configured to be placed subcutaneously utilizing a minimally invasive approach. Subcutaneous electrodes are provided on the housing 102 to simplify the implant procedure and eliminate a need for a transvenous lead system. The sensing electrodes may be located on opposite sides of the device and designed to provide robust episode detection through consistent contact at a sensor-tissue interface. The IMD 100 may be configured to be activated by the patient or automatically activated, in connection with recording subcutaneous ECG signals.

The IMD 100 senses far field, subcutaneous CA signals, processes the CA signals to detect arrhythmias and if an arrhythmia is detected, automatically records the CA signals in memory for subsequent transmission to an external device 154.

The IMD 100 is implanted in a position and orientation such that, when the patient stands, the IMD 100 is located at a reference position and orientation with respect to a global coordinate system 10 that is defined relative to a gravitational direction 12. For example, the gravitational direction 12 is along the Z-axis while the X-axis is between the left and right arms.

As explained herein, the IMD 100 includes electrodes that collect cardiac activity (CA) signals in connection with multiple cardiac beats and in connection with different IMD locations (e.g., different positions and/or different orientations). The IMD 100 also includes one or more sensors to collect acceleration signatures that are indicative of heart sounds produced at different points in a cardiac cycle.

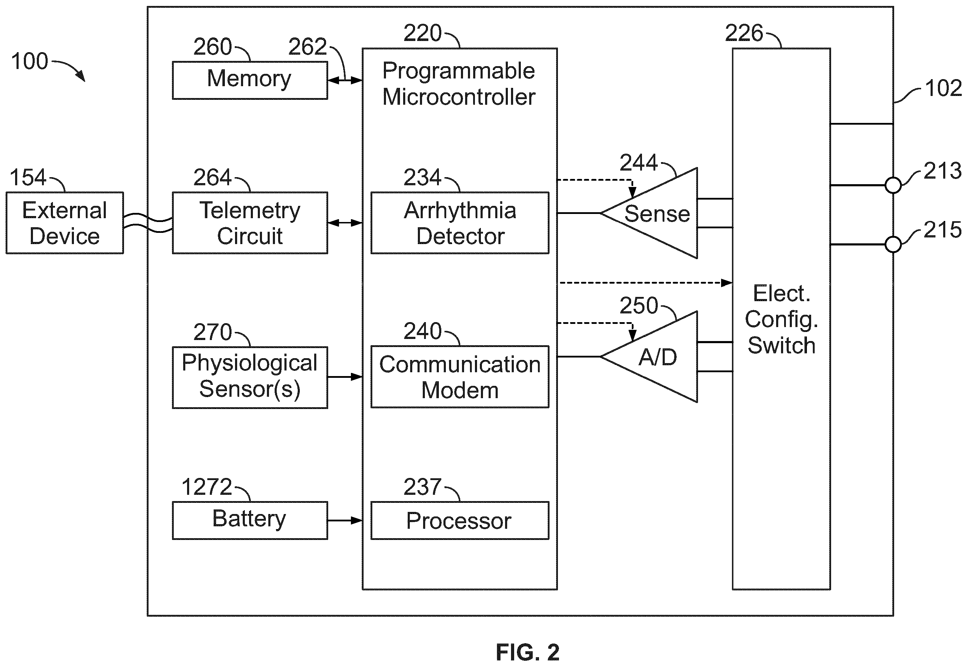

Figure 2 shows an example block diagram of the IMD 100 formed in accordance with embodiments herein. The IMD 100 may be implemented to monitor ventricular activity alone, or both ventricular and atrial activity through sensing circuit. The IMD 100 has a housing 102 to hold the electronic/computing components. The housing 102 (which is often referred to as the "can," "case," "encasing," or "case electrode") may be programmably selected to act as an electrode for certain sensing modes. Housing 102 further includes a connector (not shown) with at least one terminal 213 and optionally additional terminals 215. The terminals 213, 215 may be coupled to sensing electrodes that are provided upon or immediately adjacent the housing 102. Optionally, more than two terminals 213, 215 may be provided to support more than two sensing electrodes, such as for a bipolar sensing scheme that uses the housing 102 as a reference electrode. Additionally or alternatively, the terminals 213, 215 may be connected to one or more leads having one or more electrodes provided thereon, where the electrodes are located in various locations about the heart. The type and location of each electrode may vary.

The IMD 100 includes a programmable microcontroller 220 that controls various operations of the IMD 100, including cardiac monitoring. Microcontroller 220 includes a microprocessor (or equivalent control circuitry), RAM and/or ROM memory, logic and timing circuitry, state machine circuitry, and I/O circuitry. Microcontroller 220 includes an arrhythmia detector 234 that is configured to analyze the far field cardiac activity signals to identify the existence of an arrhythmia. The microcontroller 220 also includes arrhythmia determination circuitry 234 for analyzing the CA signals to assess a presence or absence of R-waves within the cardiac beats from a first segment of the CA signals and detect an arrythmia based on the presence or absence of one or more R-waves from the cardiac beats within a second segment of the CA signals.

The microcontroller 220 also includes an HS signal analysis (HSA) processor 237. The HSA processor 237 is configured to implement one or more of the operations discussed herein. The HSA processor 237 is configured to be a computer implemented method to identify a characteristic of interest (COI) of a heartbeat from the CA signals and overlay a HS search window onto an HS segment of the HS signals based on the COI from the CA signals. The HSA processor 237 can make determinations related an EMAT or sound data values based on the COI of the heartbeat and HSs. In one example the HSA processor 237 can calculate a center of mass (COM) for at least one of S1 or S2 HS based on the HS segment of the HS signals within the search window to obtain a corresponding at least one of S1 COM or S2 COM, and calculate at least one of EMAT or SI data values based on the at least one of S1 COM or S2 COM. The microcontroller 220 records at least one of the EMAT or SI data over time to form an EMAT trend and in SI trend.

In an example, the HSA processor 237 is configured to overlay the S1 and S2 search windows over corresponding HS segments. The HSA processor 237 is configured to align the S1 search window over the HS signals to begin at or near an R-wave peak, the R-wave peak representing the COI. The HSA processor 237 is configured to align the S2 search window over the HS signals to begin a predetermined interval after one of an end of the S1 search window or an R-wave peak, the R-wave peak representing the COI. The HSA processor 237 is configured to calculate the S1 COM by: calculating products of i) amplitudes of the HS signals at points along the S1 search window and ii) positions of the corresponding points along the S1 search window; summing the products to form a first sum; summing the amplitudes of the HS signals at the points to form a second sum; and dividing the first sum by the second sum. As explained herein, the S1 COM and S2 COM represent corresponding points in time along the CA and HS signals. The COI occurs at a COI point in time along the CA signals, the one or more processors configured to calculate the EMAT by subtracting the S1 COM from the COI point in time. The HSA processor 237 is configured to calculate the SI as a difference between the S1 COM and the S2 COM.

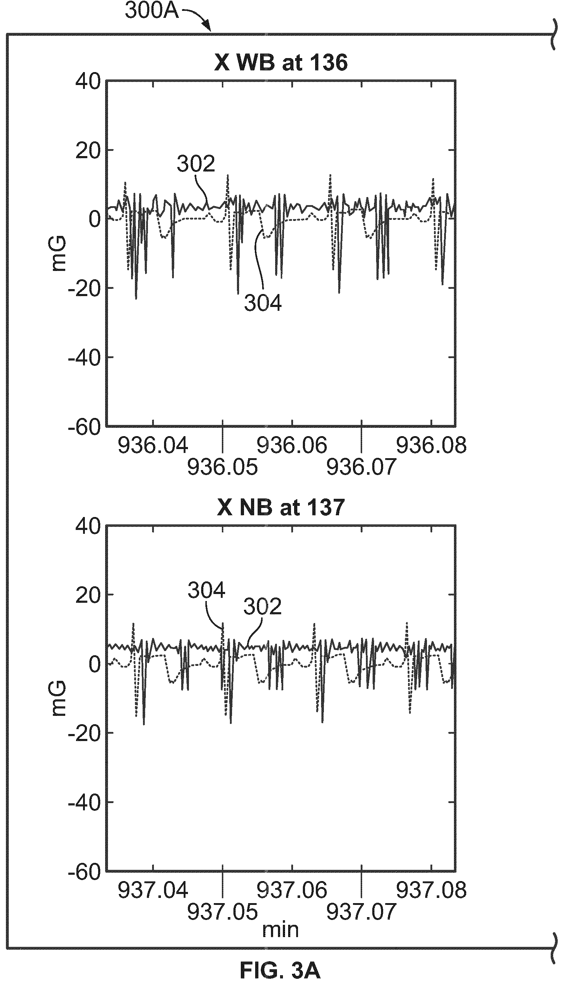

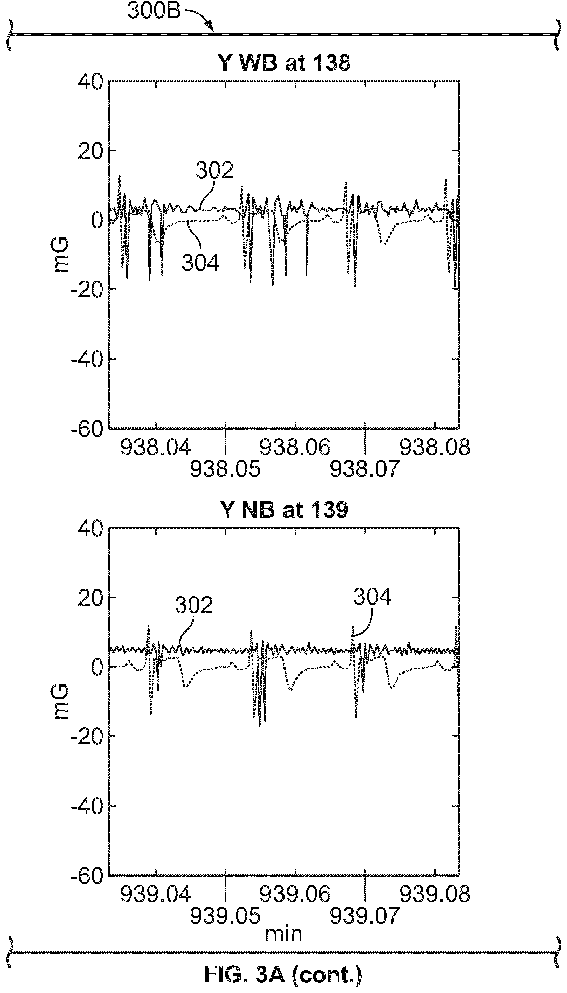

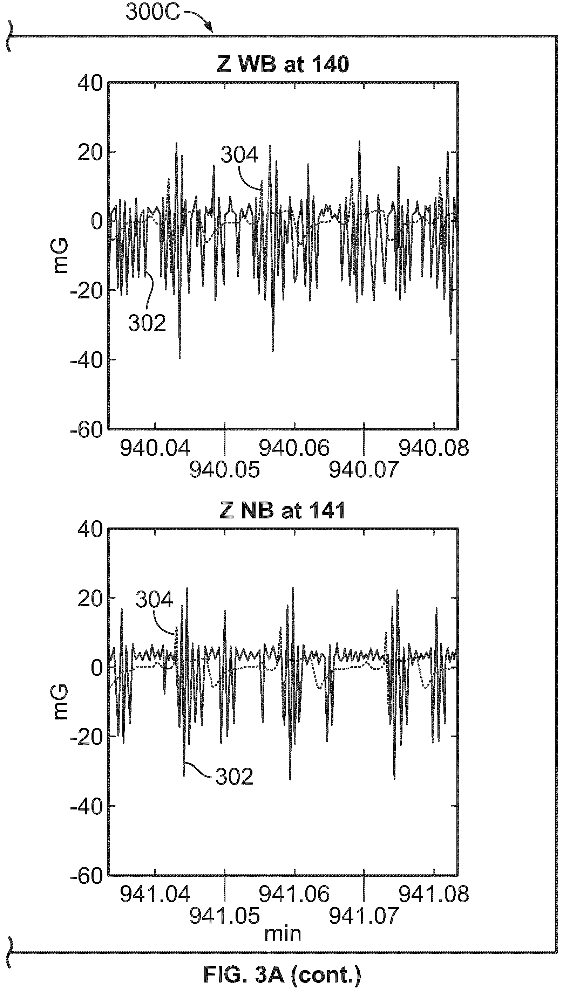

In another example, the HSA processor 237 obtains the HS data collected from a physiologic sensors 270 that is a 3D AXL related to S1, S2, and S3 sounds that represent various parts of the cardiac cycle. Figure 3A illustrates example graphs 300A-300C of raw HS data (signal) 302 overlaid on top of a surface ECG signal 304. Each set of graphs can represent different HS frequency components for each axis of a HS sensor (e.g., 300A represents an X-axis wide bandwidth and X-axis narrow bandwidth, 300B represents a Y-axis wide bandwidth and Y-axis narrow bandwidth, and 300C represents a Z-axis wide bandwidth and a Z-axis narrow bandwidth). As illustrated, the average S1 and S2 peak-to-peak amplitudes can range from approximately 16 - 31 mGs when measured by the 3D AXL placed near the heart as in typical ICM placement. The S3 peak-to-peak amplitudes can be in the range of 2 - 8 mGs. The range of heart sound amplitudes may vary depending on the location of device implant relative to the heart.

For HS data collection by the IMD that provides the HS data for Fig. 3A, HS data is collected to output all six bandwidth HS components (X-axis wide bandwidth and narrow bandwidth 300A, Y-axis wide bandwidth and narrow bandwidth 300B, and Z-axis wide bandwidth and narrow bandwidth 300C). Alignment of the bandwidth HS components with R-waves provides important timing information. The alignment can be accomplished by storing ensemble averages of multiple HSs aligned on the sensed ventricular event along with indication of the sensed event in the raw heart sound waveform.

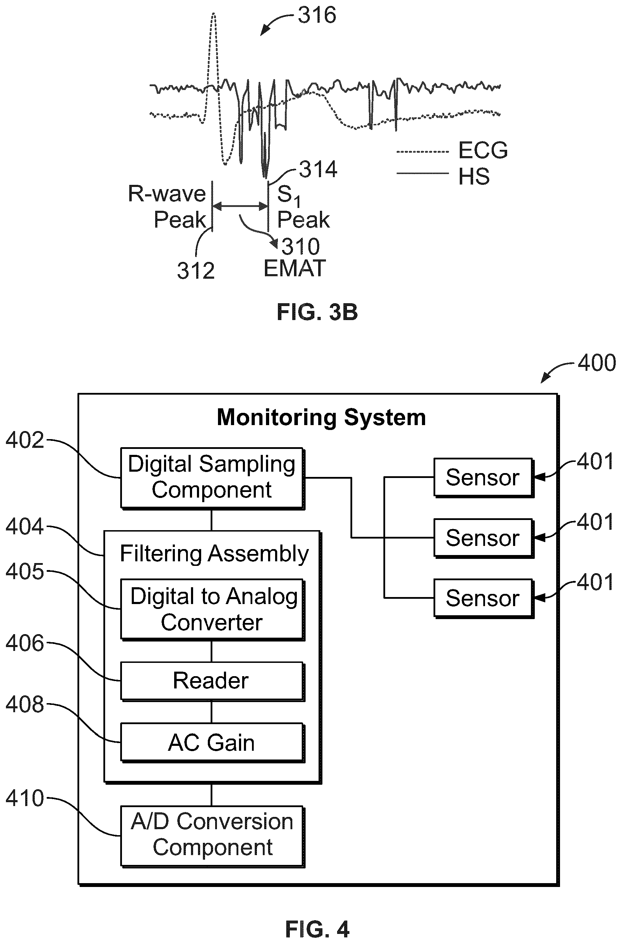

In one example, temporal measurement bandwidth HS component(s) can be used in measuring EMAT or other timing intervals for a cardiac cycle. In an example shown in Figure 3B, EMAT 310 can be measured as an interval from the peak 312 of the QRS complex of an ECG signal to the S1 peak 314. The electrical component of the EMAT cycle may be derived from other fiducial points such as the onset of the QRS complex 316, a ventricular sensing/pacing event marker, or even atrial marker/detection of P wave, to the S1 heart sound component 314 within the same cardiac cycle.

With reference back to Figure 2, in accordance with embodiments herein, the microcontroller 220 manages storage of the EMAT and SI over a period of time and monitors an EMAT trend and an SI trend over the period of time for an indication of a change in a physiologic or non-physiologic condition. For example, the microcontroller 220, also referred to as an IMD processor, may be configured to perform all or more than one of the identify, overlay, or calculate operations. The external device 154 is configured to wireless communicate with the IMD 100. The external device 154 includes ED memory and one or more ED processors. The one or more ED processors may be configured to perform at least one of the identify, overlay, and calculate operations. As one example, the external device 154 may wirelessly receive the CA and HS signals, and the one or more ED processors may perform the identify, overlay and both calculate operations.

Although not shown, the microcontroller 220 may further include other dedicated circuitry and/or firmware/software components that assist in monitoring various conditions of the patient's heart and managing pacing therapies. A switch 226 is optionally provided to allow selection of different electrode configurations under the control of the microcontroller 220. The switch 226 is controlled by a control signal 228 from the microcontroller 220. The IMD 100 may be further equipped with a communication modem (modulator/demodulator) 240 to enable wireless communication. In one implementation, the communication modem 240 uses high frequency modulation, for example using RF, Bluetooth or Bluetooth Low Energy telemetry protocols. The signals are transmitted in a high frequency range and will travel through the body tissue in fluids without stimulating the heart or being felt by the patient. The communication modem 240 may be implemented in hardware as part of the microcontroller 220, or as software/firmware instructions programmed into and executed by the microcontroller 220. Alternatively, the modem 240 may reside separately from the microcontroller as a standalone component. The modem 240 facilitates data retrieval from a remote monitoring network. The modem 240 enables timely and accurate data transfer directly from the patient to an electronic device utilized by a physician.

The IMD 100 includes sensing circuit 244 selectively coupled to one or more electrodes that perform sensing operations through the switch 226 to detect CA data indicative of cardiac activity. The sensing circuit 244 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the features of interest. In one embodiment, switch 226 may be used to determine the sensing polarity of the CA signal by selectively closing the appropriate switches. The IMD 100 further includes an analog-to-digital A/D data acquisition system (DAS) 250 coupled to one or more electrodes via the switch 226 to sample CA signals across any pair of desired electrodes. The HSA processor 237 may be applied to signals from the sensing circuit 244 and/or the DAS 250.

By way of example, the external device 154 may represent a bedside monitor installed in a patient's home and utilized to communicate with the IMD 100 while the patient is at home, in bed or asleep. The external device 154 may be a programmer used in the clinic to interrogate the IMD 100, retrieve data and program detection criteria and other features. The external device 154 may be a handheld device (e.g., smartphone, tablet device, laptop computer, smartwatch, and the like) that may be coupled over a network (e.g., the Internet) to a remote monitoring service, medical network, and the like. The external device 154 may communicate with a telemetry circuit 264 of the IMD through a communication link 266. The external device 154 facilitates access by physicians to patient data as well as permitting the physician to review real-time CA signals while collected by the IMD 100.

The microcontroller 220 is coupled to a memory 260 by a suitable data/address bus 262. The memory 260 stores the motion data, baseline motion data sets, CA signals, as well as the markers and other data content associated with detection and determination of the arrhythmia.

The IMD 100 may further include one or more physiologic sensors 270. For example, the physiologic sensor 270 can be a HS sensor and may represent one or more AXLs, such as a three-dimensional (3D) AXL. The sensor 270 may utilize a piezoelectric, a piezoresistive, and/or capacitive components commonly used to convert the mechanical motion of the 3D AXL into an electrical signal received by the microcontroller 220. By way of example, the 3-D AXL may generate three electrical signals indicative of motion in three corresponding directions, namely X, Y and Z directions. The electrical signals associated with each of the three directional components may be divided into different frequency components to obtain different types of information therefrom.

In one example the 3D AXL can include three DC sensors that can be considered superior-inferior (X-axis), anterior-posterior (Y-axis), and lateral-medial (Z-axis), respectively. Each DC sensor can also generate the activity level of the patient, i.e., AC acceleration. Each of the DC sensors is preferably a surface micromachined integrated circuit with signal conditioning as is well known in the art. Employing surface micromachining, a set of movable capacitor plates are formed extending in a pattern from a shaped polysilicon proof mass suspended by tethers with respect to a further set of fixed polysilicon capacitor plates. The proof mass has a sensitive axis along which a force between 0 G and +50 G effects the physical movement of the proof mass and a change in the measured capacitance between the fixed and moveable plates. The measured capacitance is transformed by the on-chip signal conditioning circuits into a low voltage signal. However, many other types of accelerometers are commercially available, and it would be obvious to one of ordinary skill in the art to use other types of accelerometers in place of the above-described multi-axis DC accelerometer. While in one example three or more DC sensors are utilized, in other examples systems can be formed from two DC sensors.

To determine both the activity level and the body position of the patient, a controller can monitor the output of each of the DC sensors. Preferably using standard analog to digital conversion techniques, the output of each of the DC sensors is filtered to separate an AC signal component (representing the activity level) and a DC signal component (representing the body position). Then, the two resultant signals are further processed to determine two corresponding digital outputs which represent the instantaneous signal level of each signal. The resulting activity digital signals are then further processed to determine the activity level of the patient by methods well known within the art.

In another example, the physiologic sensor 270 collects device location information with respect to gravitational force while the IMD 100 collects CA signals in connection with multiple cardiac beats. While shown as being included within the housing 102, the physiologic sensor(s) 270 may be external to the housing 102, yet still, be implanted within or carried by the patient.

Figure 4 illustrates a schematic diagram of a HS sensor (e.g., such as physiologic sensor 270) that may be implemented as an AXL, more generally referred to herein as a monitoring system 400. The monitoring system 400 is used to detect and determine heart sound signals. In one embodiment, the monitoring system 400 is a three-dimensional AXL that may be implemented as a chip for placement in an IMD. In another embodiment, the AXL is formed and operates in the manner described in US patent 6,937,900, titled AC/DC MULTI-AXIS ACCELEROMETER FOR DETERMINING A PATIENT ACTIVITY AND BODY POSITION. In yet another embodiment, the AXL is formed and operates in the manner described in US Patent Publication 2021/0350931, titled METHOD AND SYSTEM FOR HEART CONDITION DETECTION USING AN ACCELEROMETER.

The AXL can include sensors that generate first (X), second (Y) and third (Z) AXL signals along corresponding X, Y and Z axes (also referred to as first axis AXL or HS signals, second axis AXL or HS signals and third axis AXL or HS signals). The X, Y and Z AXL signals collectively define a three-dimensional, or multi-dimensional (MD) AXL or HS data set. While examples herein are described in connection with an AXL that generates AXL signals along three orthogonal axes, it is recognized that embodiments may be implemented wherein AXL signals are generated along two or more axes, including more than three axes.

Each of the first (X), second (Y) and third (Z) AXL signals can further be divided into at least first and second bandwidth frequency ranges. The first and second bandwidth frequency ranges differ from one another. For example, the first and second bandwidth frequency ranges may be mutually exclusive of one another (e.g., a lower frequency range that does not overlap a higher frequency range). Alternatively, the first and second frequency ranges may partially overlap (e.g., a lower frequency range that has an upper portion that partially overlaps a lower portion of a higher frequency range). Alternatively, the first frequency range may be a subset of the second frequency range. As an example, AXL signals can be obtained on each axis at two HS bandpass filter settings: wideband (WB), (e.g., with a frequency range of 7.5 - 100 Hz), and narrowband (NB), (e.g., with a frequency range of 15 - 100 Hz, for a total of six bandwidth HS components). A first bandwidth HS component (first/X axis within a first frequency/NB), a second bandwidth HS component (first /X axis within a second frequency/WB), a third bandwidth HS component (second/Y axis within a first frequency/NB), a fourth bandwidth HS component (second/Y axis within a second frequency/WB), a fifth bandwidth HS component (third/Z axis within a first frequency/NB), or a sixth bandwidth HS component (third/Z axis within a second frequency/WB) may be obtained from the AXL.

The monitoring system 400, or HS sensor, may include sensors 401 that monitor and receive signals from the X, Y and Z axes to obtain AXL data. In one embodiment, the individual X, Y and Z signals are received by a digital sampling component 402 that receives a digital input. Coupled to the digital sampling component 402 is a filtering assembly 404 that may include a digital to analog converter 405 to form an alternating current (AC) signal, a reader device 406, and an AC gain device 408. While in this embodiment, the filtering assembly includes the devices provided, in other examples, other devices may be utilized to filter the digital input signal for processing.

The monitoring system 400 may also include an analog to digital conversion component 410, along with a position, or direct current (DC) component. In one example, the analog to digital conversion component may be a 3-bit analog to digital converter (ADC). The evaluation version of the monitoring system 400 may provide 3-axis (X and Y along the chip, Z normal to the chip) DC-coupled posture signal corresponding to 3 orthogonal directions as well as 3-axis AC-coupled activity signal. In one embodiment, each of the 6 signals (and corresponding bandwidth HS components) may be sampled at 100 Hz and accumulated over 1 sec for a total of 12 signals([X/Y/Z], [posture/activity], [100/1 Hz]). This AXL data may be used to describe embodiments herein.

While described as a digital signal in relation to Figure 4, in other embodiments the signal may be an analog signal, filtered, amplified, etc. The AXL data signals may be recorded in a data storage of the AXL, of an IMD, of a remote device etc. Alternatively, the AXL data set may be obtained from a remote device or received from a storage device coupled to the AXL. In one example, the AXL data set may be a multi-dimensional AXL data set.

One or more embodiments generally relate to leadless IMDs and systems, such as pacemakers, implantable cardioverter-defibrillators, cardiac rhythm therapy devices and the like. As explained hereafter, embodiments utilize heart sounds to calculate one or more heart function (HF) indicators for an individual patient and to use the HF indicators to determine one or more therapy related (TR) delays and/or sensing related blanking intervals (SR Bls).

In one example, one or more processors can utilize the AXL data, including the first COI (e.g. S1 amplitude, S2 amplitude, S3 amplitude, S1 to S2 interval, S2 to S3 interval, S3 to S1 interval or the like), second COI, third COI, fourth COI, fifth COI, and sixth COI of a heartbeat from HS signals obtained over a first period of time to determine the combined axis and frequency range (e.g., the bandwidth HS component) providing the most desirable readings. The bandwidth HS component with the most desirable readings can be selected and the selected bandwidth HS component can be used for obtaining the HS data during a second period of time. The first period of time can be for a limited period of time such as thirty seconds, one minute, five minutes, ten minutes, or the like. The second period of time can be for longer, such as days, weeks, months, etc. In one example, the first period of time can be a testing period of time and identifying COls from as many different bandwidth HS components as possible. The best possible bandwidth HS component for a COI can be selected for use during the second period of time. In one example, a custom, or individualized determination can be provided for each individual user.

In one example, the first period of time can be broken up into different intervals. As an example, during a first interval, a first COI is identified at a first axis (X, Y, or Z) and within a first frequency range (NB or WB) (e.g., a first bandwidth HS component), during a second interval that is sequential, or after the first interval, a second COI can be obtained using a second bandwidth HS component. In an example when three axis and two frequencies are present, six successive intervals can occur one after another during the first period. After the first six intervals are taken, additional intervals can occur until enough information is obtained to determine the best combination for obtaining the COI.

In one embodiment, the selection of the bandwidth HS component is based on an ensemble average of multiple R-wave-aligned heart sounds collected over a collection period for each HS sensor configuration for a series of beats. The alignment of R-waves can be either from the start of the QRS complex or from the peak of the R-wave. A raw heart sound waveform snippet for each bandwidth HS component, can be taken from the same measurement as the ensemble average, with an indication of position of the sensed event.

Because the HS waveforms are different in each of the bandwidth HS component, each bandwidth HS component has a separate ensemble average. Additionally, each data collection session needs to have its own set of ensemble averages, rather than adding on to the previously collected data, because the heart rate or patient position may differ from session to session. In one example the changing of heart rate or patient position can change the morphology or timing of heart sounds. A histogram of the distribution of the intervals used in calculating each ensemble average is useful in gauging the quality of the ensemble average and the underlying HS.

For example, raw HS data can be available for an extended period of time, or interval, that in one example can be at least one minute. The COI can be obtained, or extracted, for every beat during that extended period (e.g., one minute). By obtaining the COI for every beat over the extended period of time sixty to eighty COI measurements can be obtained (based on the number of beats per minute of the patient).

For example, when measuring a first axis within a first bandwidth sixty (60) S1 amplitudes (e.g., the COI), can be obtained during a first interval (e.g., the extended period of time). At this time, a standard deviation of the COIs can be measured (e.g., a SD of the 60 S1 amplitudes). In this example, if the HS signal is stable, the 60 S1 amplitudes are relatively stable with a low standard deviation. For example, when taking the coefficient of variability (cov), which is standard deviation of S1 / average of S1, if the cov is less than (<) a threshold (i.e., 10%) an indication is provided that the axis and bandwidth are acceptable for obtaining the HS signals for making determinations. In one example, a standard deviation can be determined for the COI obtained for each bandwidth HS component (e.g., X-axis within a low bandwidth, X-axis within a high bandwidth, etc.) using the same methodology (taken for each beat over an extended period of time such as a minute). In such an embodiment, the bandwidth HS component with the lowest standard deviation can be selected as the axis and bandwidth (e.g., bandwidth HS component) to be utilized for obtaining COIs moving forward. Alternatively, each bandwidth HS component can be determined sequentially (e.g., the first axis and first bandwidth first, the first axis and second bandwidth second, the second axis and first bandwidth third, etc.) until a standard deviation is below the threshold standard deviation (e.g., below 10%). Upon determination that a bandwidth HS component meets this standard, that bandwidth HS component may be utilized to obtain COls moving forward during a second period without obtaining additional COls from other bandwidth HS components.

Similarly, in another example, an ensemble average signal for the extended period can be obtained for each bandwidth HS component. The covariance (mean value of the product of the deviation of variates from their respective means) of the ensemble average against the HS signal is obtained for each beat during the extended period. If the average covariance is greater than a threshold (i.e., .5) the bandwidth HS component is considered acceptable. Similar to the standard deviation, in one example the average covariance can be determined for all bandwidth HS components and the greatest average covariance can be selected, whereas alternatively, once an acceptable average covariance is determined that bandwidth HS component is utilized thereafter.

In yet another example, RR intervals for every beat of an extended period of time can be obtained. So, in an example when a patient's heart beats 60 times in a minute, fifty-nine (59) RR intervals are obtained during an extended period of time of one minute. Because ensemble average is derived from using a fiducial point from QRS, if the heart rate is unstable, the resultant ensemble average may not be derived from a consistent HS signal. So by evaluating the RR interval stability and whether greater certain stability criteria such as covariance being less than .1 (10%), the HS measure can be considered acceptable. So again, either the bandwidth HS component with the best interval stability can be selected, or once an acceptable bandwidth HS component is determined that bandwidth HS component can be utilized thereafter. In addition, standard deviations, covariances of ensemble averages, or the like can be utilized for other COls to determine a bandwidth HS component to be utilized.

In each example, a COI is obtained for each beat of a series of beats over the extended period of time. COIs obtained are compared to one another to determine a variance between the COls obtained. In yet another example, the variance can be represented by a standard deviation, a covariance, or the like to determine whether a bandwidth HS component meets a determined standard or threshold. If the threshold is met, the bandwidth HS component being analyzed can be utilized for sensing HS.

Optionally, each bandwidth HS component can be analyzed during sequential intervals of the first period, where each interval represents a period of time COI data is being obtained for a bandwidth HS component (e.g., first axis within a first bandwidth, etc.) and the bandwidth HS component with the best results can be selected for obtaining the COI data during a second period. Alternatively, each bandwidth HS component can be analyzed during sequential intervals of the first period until an acceptable bandwidth HS component is determined. After the acceptable bandwidth HS component is determined that bandwidth HS component is utilized during the second period and no additional HS data is obtained during the first period. So, if a first variance of a first COI of a first bandwidth HS component is deemed not acceptable after the first COls are analyzed, a set of second COIs for a second bandwidth HS component over a second interval during the first period are obtained and analyzed for a second variance. As used herein, the term "second" in the phrase "second COI" does not mean a different COI is being obtained compared to the "first COI", instead the term "second" indicates that the COI is being obtained based on the second bandwidth HS component. In one example, the first COI may be an S1 amplitude related to the first bandwidth HS component while the second COI may be an S1 amplitude related to the second bandwidth HS component. So, after the second COls are analyzed to determine a second variance, if the second variance of the second bandwidth HS component to deemed acceptable, the second bandwidth HS component is utilized during the second period with no additional COIs being obtained or analyzed during the first period. Whereas if the second variance related to the second bandwidth HS component is deemed not acceptable, third COls are obtained and analyzed during a third interval of the first period and so on until an acceptable bandwidth HS component is determined.

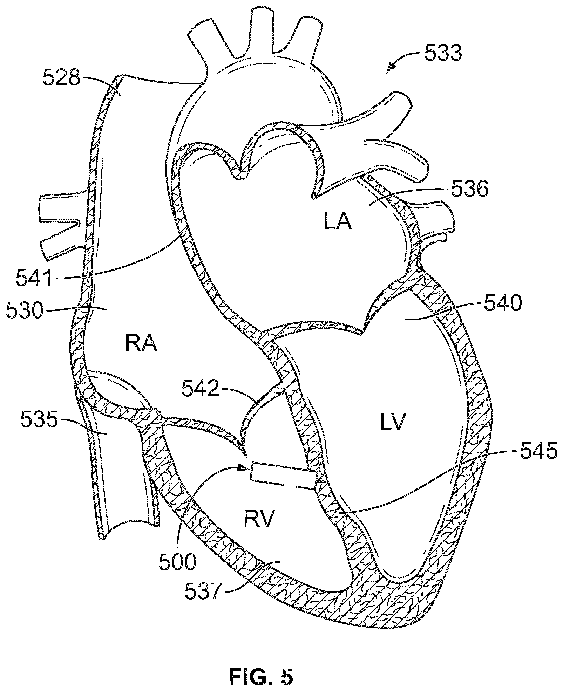

Figure 5 provides a sectional view of a patient's heart 533 and shows a leadless implantable medical device (IMD) 500. The IMD 500 has been placed through the superior vena cava 528 into the right atrium 530 of the heart 533. Figure 5 also shows the inferior vena cava 535, the left atrium 536, the right ventricle 537, the left ventricle 540, the atrial septum 541 that divides the two atria 530, 536, and the tricuspid valve 542 between the right atrium 530 and the right ventricle 537.

The IMD 500 is formed in accordance with an embodiment. The IMD 500 may represent a pacemaker, a cardiac resynchronization therapy (CRT) device, a cardioverter, a cardiac rhythm management (CRM) device, a defibrillator, or the like. The IMD 500 comprises a housing 502 configured to be implanted entirely within a single local chamber of the heart 533, such as entirely and solely within the right atrium 530, left atrium 536, the right ventricle 537 or the left ventricle 540, for example. Optionally, the IMD 500 may be implanted outside of the chambers of the heart but located in a vessel proximate to, or attached to an exterior wall of, the heart proximate to the RA, LA, RV or LV.

The chamber in which the IMD 500 is implanted in (or closest proximally to) is referred to as the "local" chamber. The local chamber includes a local chamber wall that is physiologically responsive to local activation events originating in the local chamber. The local chamber is at least partially surrounded by local wall tissue that forms, contains, or constitutes at least part of a conduction network for the associated chamber.

As shown in Figure 5, the local chamber in which the IMD 500 is implanted is the right ventricle 537. For example, the IMD 500 is mounted or fixated to the tissue wall of the right ventricle 537 along the septum 545 that divides the right ventricle 537 from the left ventricle 540. The septum 545 wall tissue in the right ventricle 537 may behave physiologically differently than the non-septum ventricular wall tissue. Optionally, the IMD 500 may be implanted in other regions of the RV, in other chambers of the heart, in vessels along an exterior of a local chamber or implanted in the exterior wall of the heart proximate to a local chamber (e.g., through the epicardium and into the myocardium). Figure 5 shows the IMD 500 in the septal wall of the RV, but optionally, the IMD 500 may be implanted at a higher location proximate to the HIS bundle in the RV. Optionally, the IMD 500 may be implanted in the RA or elsewhere. Alternatively, multiple IMDs may be implanted into the patient's heart 533 within different chambers or different segments of the same chamber.

The leadless IMD 500 may sense various intrinsic events and deliver corresponding therapies depending upon whether a subsequent intrinsic event is detected within a certain time period. For example, the IMD may utilize one or more atrial-ventricular (AV) delays to manage ventricular pacing in the event an intrinsic ventricular event does not occur within a programmed time period following a preceding intrinsic (or paced) atrial event. Similarly, the IMD may utilize one or more ventricular-ventricular (VV) delays to manage synchronization between right and left side ventricular activity. For example, a leadless IMD in the RV may deliver a paced event when an intrinsic right side ventricular event does not occur within a programmed time period following a preceding intrinsic (or paced) ventricular event in the left ventricular chamber, or vice versa. As another example, the IMD may utilize one or more atrial-HIS (AH) delays to manage HIS bundle pacing in the event an intrinsic event does not occur at the HIS bundle within a programmed time period following a preceding intrinsic (or paced) atrial event. As another example, when the IMD is implanted in the atrium, the IMD may utilize one or more post ventricular atrial refractory period (PVARP) blanking intervals to manage blanking for a sensing circuit following a preceding intrinsic ventricular event.

As explained herein, the leadless IMD "listens" for and detects intrinsic events based on one or more heart sounds of interest. The HS of interest is used to start one or more TR delays and/or SR blanking intervals. The TR delay and/or SR blanking interval is calculated in part based on COM calculations for one or more heart sounds.

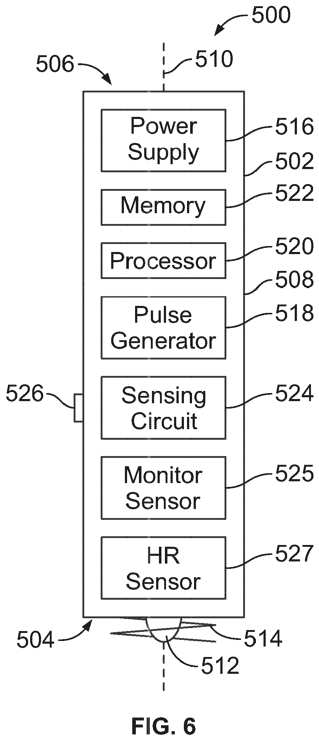

Figure 6 illustrates a side view of the IMD 500 according to an embodiment. The illustrated IMD 500 includes a schematic representation of some internal components of the IMD 500. The housing 502 of the IMD 500 includes a first mounting end 504, an opposite second end 506, and an intermediate shell 508 extending between the first end 504 and the second end 506. The shell 508 is elongated and tubular in shape and extends along a longitudinal axis 510. The mounting end 504 mounts to tissue of an intra-cardiac wall within a chamber of the heart.

The mounting end 504 includes an electrode 512 securely attached thereto and projecting outward from the mounting end 504. The shell 508 includes one or more electrodes 526 provided therein remote from the electrode 512. The electrodes 512 and 526 cooperate to define a sensing vector and to sense local CA signals. The electrodes 512 and 526 are further configured to deliver stimulation energy to tissue of interest. As used herein, "tissue of interest" refers to intra-cardiac tissue that the IMD 500 is configured to monitor and provide stimulation energy. In the illustrated embodiment, the IMD 500 is configured to be affixed directly to the tissue of interest, as described below. The electrode 512 may be a cathode electrode that is actively fixated to the myocardium, while the electrode 526 is an anode electrode. The stimulation energy may be in the form of low-energy pacing pulses, higher-energy shocking pulses, or the like.

When the mounting end 504 is mounted to the intra-cardiac tissue, the electrode 512 is securely affixed to and engages the tissue of interest to deliver the stimulation energy directly thereto. In addition to delivering stimulation energy, in an alternative embodiment the electrode 512 may be used to sense electrical activity from the tissue of interest. The electrode 512 may be formed as a single conductive bulb or, alternatively, as a cone, a single wire, or the like. Optionally, the electrode 512 is not covered with insulation material and the conductive material is exposed to facilitate a good electrical connection to the local wall tissue. Alternatively, at least a portion of the electrode 512 is covered with insulation to prevent electrical conduction to tissue that engages the insulation.

The mounting end 504 includes a fixation element to secure the IMD in or proximate to a local chamber of the heart. For example, the fixation element may be a fixation screw 514 securely attached thereto and projecting outward from the mounting end 504. The fixation screw 514 is configured to extend into the tissue of interest to anchor the IMD 500 to the intra-cardiac tissue. The fixation screw 514 is configured to be screwed into the tissue to firmly adhere the IMD 500 thereto by pressing the mounting end 504 against the tissue and rotating the IMD 500 in a first, coupling direction. The fixation screw 514 may be extracted from the tissue by rotating the IMD 500 in an opposite, uncoupling direction in conjunction with a slight tugging force directed away from the myocardial wall. The fixation screw 514 may be shaped as a helical corkscrew that defines a center channel. For example, the fixation screw 514 may surround the electrode 512 such that the electrode 512 is within the center channel. In an alternative embodiment, the fixation screw 514 is part of the electrode 512. For example, the electrode 512 may have helical threads on an outer surface of the electrode 512, such that the electrode 512 forms the fixation screw 514.

Additionally or alternatively, the fixation element may include one or more loops, tabs and the like that are configured to retain the IMD in a vessel, septal wall, or other tissue proximate to the local chamber of the heart. For example, the IMD and/or fixation element may be formed as described in one or more of U.S. Pat. Pub. No. 2019/0099087, titled WIRELESS SENSOR FOR MEASURNG PRESSURE; U.S. Patent 9,993,167, titled APPARATUS AND METHOD FOR SENSOR DEPLOYMENT AND FIXATION; U.S. Published Application 2016/0007924, titled IMPLANTABLE PRESSURE TRANSDUCER SYSTEM OPTIMIZED TO CORRECT ENVIRONMENTAL FACTORS.

The housing 502 retains a power supply 516 and various electronic components that receive electrical current from the power supply 516. The electronic components provide the functionality of the IMD 500, such as controlling the stimulation energy delivered to the electrode 512 and sensing the depolarization along the tissue of interest in response to a pacing pulse or to an intrinsic heartbeat. The power supply 516 stores charge for gradual disbursal to the electronic components as needed. The power supply 516 may be a battery. The power supply 516 has a fixed amount of charge at full capacity. The power supply 516 may be rechargeable in some embodiments and may not be rechargeable in other embodiments. The power supply 516 is fully retained within and surrounded by the housing 502.

The electronic components include a pulse generator 518, a processor 520, a memory 522, a sensing circuit 524 and a monitoring sensor/system 525, such as monitoring system 400 (Figure 4) and/or physiologic sensor 270 (Figure 2). The illustration is intended as an overview of the electronic components only, and the electronic components according to an embodiment of the IMD 500. The pulse generator 518 provides stimulation energy to the electrode 512 which is delivered to the tissue of interest that the electrode 512 engages. The pulse generator 518 includes circuitry to control the output of stimulation energy directed to the electrode 512. For example, the pulse generator 518 produces lower energy pulses for pacing and higher energy pulses for shocking.

The processor 520 is a controller that controls the flow of charge between the power supply 516, the electronic components (such as the pulse generator 518, monitoring sensor 525 and the sensing circuit 524), and the electrodes (such as electrode 512). For example, the processor 520 controls the timing and intensity or magnitude of the stimulation pulses. If multiple electrodes are used to deliver stimulation energy to the intra-cardiac tissue, the processor 520 may synchronize the delivery of the pulses. The processor 520 is communicatively coupled to the pulse generator 518, the sensing circuit 524, the memory 522, and the power supply 516. The processor 120 also functions based on instructions stored locally in the memory 522. The memory 522 is a nontransitory tangible computer readable storage medium. The memory 522 stores programmable and executable instructions for the processor 520. The processor 520 is responsive to the programmable instructions to control operation of the IMD 500 as described herein. The memory 522 may also store data. Some of the data may be stored prior to completing assembly of the IMD 500, while other data may be stored during use of the implanted IMD 500. For example, the memory 522 may be used to store data on intrinsic electrical activity within the heart as monitored by the sensing circuit 524, data on the number, time, and/or magnitude of pacing pulses generated by the pulse generator 518, or the like. The memory 522 is further configured to store HS signals, S1 COMs, S2 COMs, S3 COMs, S4 COMs, EMATs, Sls, Dls, and the like.

The sensing circuit 524 is configured to monitor intrinsic electrical CA signals within the heart. The sensing circuit 524 is communicatively coupled to one or more sensing electrodes 512, 526 located on or extending from the housing 502. The sensing electrodes 526 are shown located along one or more sides of the shell 508 but may additionally or alternatively be located along the second end 506 of the housing 502. The sensing electrode 526 senses electrical activity, such as physiologic and pathologic behavior and events, and provides sensed signals to the sensing circuit 524 in response. In an alternative embodiment, the pulsing electrode 512 doubles as a sensing electrode, such that the pulsing electrode 512 is used to deliver stimulation pulses and, in-between pulses, monitors the electrical activity within the tissue of interest for the sensing circuit 524.

Optionally, the IMD 500 may include a heart rate (HR) sensor 527 configured to obtain HR data indicative of a patient's heart rate. For example, the HR sensor 527 may sense a blood temperature indicative of a core body temperature of the patient. The processor 520 is further configured to produce a relative temperature signal based on the blood temperature signal. The processor 520 further produces a moving baseline temperature signal based on the relative temperature signal, produces a proportional response signal based on the relative temperature signal and the moving baseline temperature signal, and produces a sensor indicated rate response signal based on the proportional response signal and a base rate. The sensor indicated rate response signal can also be based on a dip response signal and/or a slope response signal. When in a therapy mode, the processor 520 is configured to adjust the at least one of the TR delay or SR BI based on the HR data. For example, the processor 520 may be configured to adjust one or more pacing parameters to control a pacing rate based on the sensor indicated rate response signal. For example, the processor 520 may adjust the AV delay, VV delay, PVARP blanking period and the like, based on the sensor indicated rate response signal.

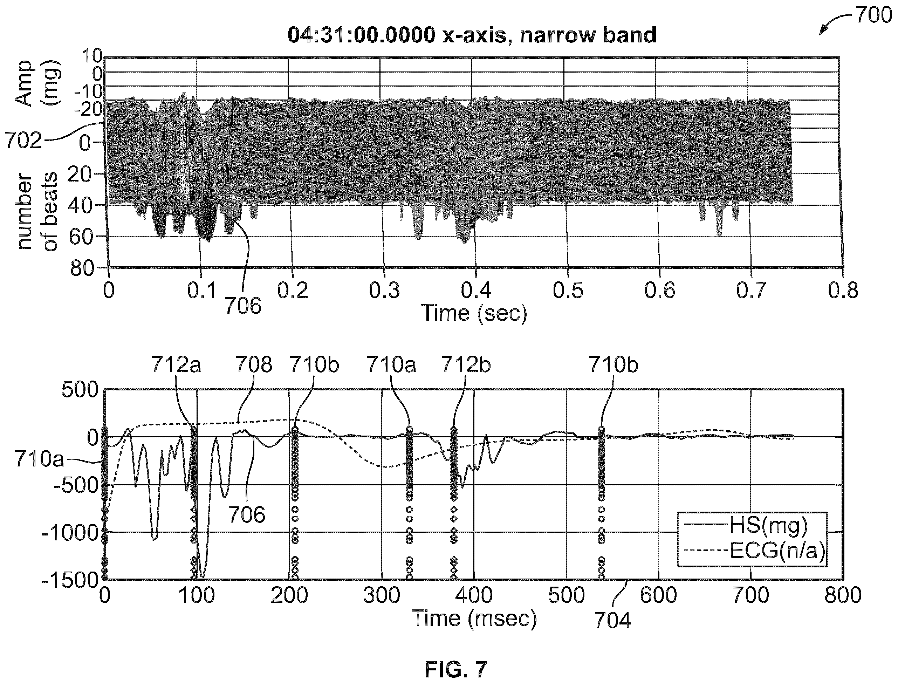

Figure 7 illustrates a graph 700 showing beats 702 over time 704 for one minute worth of beats to provide a HS signal 706 (top panel) and an ensemble average of the HS and ECG signal 708 (bottom panel). A search window 710a, 710b for S1 and S2 respectively are provided along with COM values 712a, 712b of S1 and S2.

In the COM approach using all axes of a 3D AXL as illustrated by Figure 7 takes advantage of the ability of the 3D AXL while in HS mode to collect HS data regardless the implant orientation of the ICM or other implantable devices. In this example the 3D AXL can be configured to collect signal for 60 sec from each axis and bandwidth sequentially. The signal can be analyzed to extract the HS components. Figure 7 shows an example of ensemble averaging and calculation of center of mass (COM). This method can be used to extract other HS components such as the peak of S1 or the dS1/dtmax.

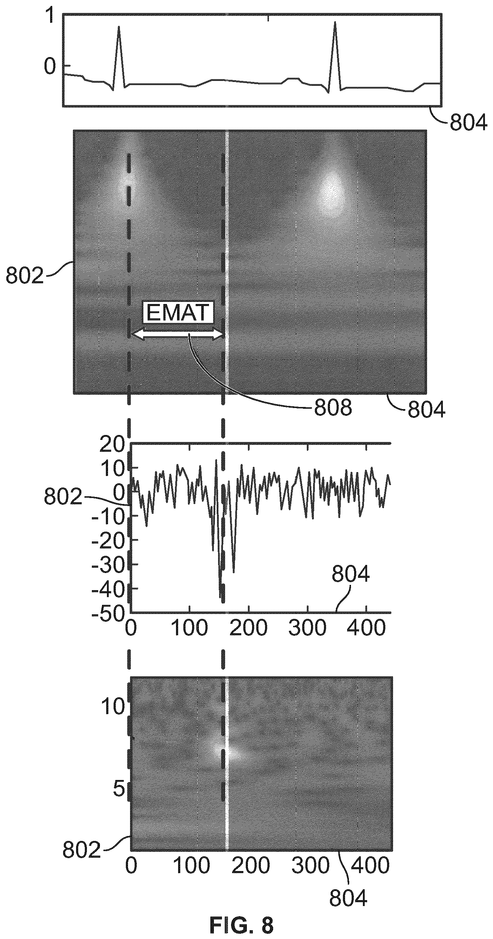

Figure 8 illustrates yet another graph for accomplishing a method for determining EMAT using a 3D AXL. The graph illustrates frequency 802 over time 804. On occasion, the method of detecting the peak, or the COM of a HS component may not be either easy to identify due to the vibratory nature of HS signal or the device may have migrated from the initial implanted position. A heart sound window may contain multiple peaks, or the intensity of the peaks may vary substantially between cardiac cycles. For example, a physician after the implantation of an ICM that has the ability to collect HS data may not be sure of his choice of HS components due to the multiple peaks of the HS signal. In this case he can activate a scalogram algorithm for the detection of EMAT for a specific axis or for all axis and bandwidths.

The scalogram algorithm can analyze the ensemble average of the collected HS data together with the ECG data. In one example Figure 8 illustrates the intensity of frequency component 802 in the time domain 804. The timing interval 808 of EMAT can be calculated as the time difference between the scalogram of the HS signal minus the scalogram of the ECG signal. In another example a migration algorithm detects a device migration can detect the scalogram algorithm to avoid any false detection of HS components due to change of the orientation of the ICM. In one example, the migration algorithm and system and related devices as described herein is at least one of the migration algorithms, systems, and related devices illustrated and escribed in U.S. Patent Publication No. 2023/0346258, entitled SYSTEM FOR DETERMINING CHANGE IN POSITION OF AN IMPLANTED POCKET. In another example embodiment, an artificial intelligence algorithm is utilized to determine migration or as part of the scalogram algorithm. The user can also program IMD remotely if the scalogram algorithm can be applied to just one specific axis and bandwidth or to all of them.

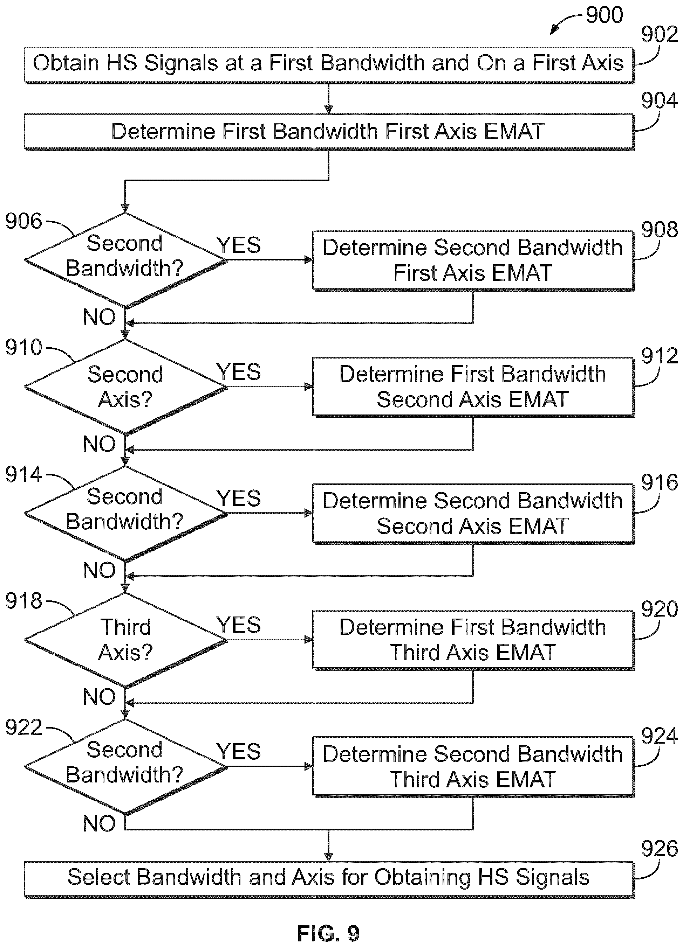

Figure 9 illustrates a method 900 for determining an EMAT utilizing HS data. In one example, the method is implemented by the systems, devices, sensors, etc. of Figures 1-6. The method 900 is performed to so that one or more processors of and IMD can determine the components of a HS sensor to utilize to obtain HS data. In one example, the IMD can obtain HS data over two bandwidths and includes a HS sensor that is a three-axis AXL that obtains HS signals along a first axis, second axis and a third axis. In an example, an EMAT determination can be made related to each bandwidth and each axis of the three-axis AXL (e.g., the bandwidth HS components). Therefore, six different EMAT determinations can be made. In other examples, the IMD may be able to obtain only one bandwidth, more than two bandwidths, only one axis, two axes, etc. Consequently, the number of EMAT determinations that can be made by the one or more processors can depend on the type of IMD and HS sensor used to obtain the HS signals. Still, once each possible EMAT determination is made, one can be selected as the selected EMAT such that additional HS signals from that point forward are obtained based on the selected EMAT. So, if a bandwidth HS component represents a first bandwidth third axis, and that bandwidth HS component results in the best EMAT results, the bandwidth HS component representing the first bandwidth third axis is utilized to obtain the additional HS signals.

At 902, a HS sensor senses and obtains HS signals within a first bandwidth and along a first axis over a period of time. In one example, the HS sensor is a three-axis AXL, and the first axis is an X-axis. In another example, the first bandwidth is a wideband bandwidth that can have a frequency range of between 7.5-100Hz. At 904, one or more processors determine a first EMAT based on the HS signals sensed.

At 906, the one or more processors determine whether the HS signals can be obtained along the first axis within a second bandwidth. In one example the second bandwidth is a narrowband bandwidth with a frequency range of between 15-100Hz. If the HS signals can be obtained along the first axis within a second bandwidth, at 908 the one or more processors obtain the HS signals on the first axis within the second bandwidth and determine a second EMAT.

If at 906, the one or more processors determine the HS signals cannot be obtained within the second bandwidth, or after the HS signals along the first axis within the second bandwidth are obtained, at 910, the one or more processors determine whether HS signals can be obtained within the first bandwidth along a second axis. If the HS signals can be obtained within the first bandwidth along the second axis, at 912, the one or more processors determine a third EMAT.

After the first EMAT is determined, at 914, the one or more processors determine whether the HS signals can be obtained within the second bandwidth along the second axis. At 916 the HS signals within the second bandwidth along the second axis are obtained, and the one or more processors determine a fourth EMAT.

After the fourth EMAT is determined, at 918 the one or more processors determine whether the HS signals can be obtained within the first bandwidth on a third axis. If the HS signals can be obtained within the first bandwidth on the third axis, at 920, the one or more processors determine a fifth EMAT.

After the fifth EMAT is determined, at 922 the one or more processors determine whether the HS signals can be obtained within a second bandwidth along the third axis. If the HS signals can be obtained within the second bandwidth along the third axis, at 924, the one or more processors determine a sixth EMAT.

After HS signals are obtained at all frequencies, and on all axes, the EMATs that are determined (e.g., the first EMAT, the second EMAT, the third EMAT, the fourth EMAT, the fifth EMAT, and/or the sixth EMAT) are analyzed, compared with one another, or the like and at 926, the one or more processors select one of these EMATs to provide a selected EMAT. In one example a shared COI of the EMATs is analyzed such as an ensemble average of the collected HS data that can be analyzed together with the ECG data. Using the shared COI the intensity of a frequency component in a time domain can be determined. The timing interval of EMAT can be calculated as the time difference between a scalogram of the HS signal minus the scalogram of the ECG signal. In another example if a migration algorithm detects a device migration, use of a scalogram algorithm can avoid any false detection of HS components due to change of the orientation of the ICM. Alternatively, a user can also have a clinician remotely program the ICM if the scalogram algorithm can be applied to just one specific axis and bandwidth or to all of them. In addition, the method of determining EMAT can be applied to all axis and frequency ranges and the optimal combination of axis and frequency range can be selected based on a shared characteristic of the EMATs. Shared characteristics of the EMATs can include S1 amplitude, highest peak frequency, S2 amplitude, signal to noise ratio, etc. The shared characteristic can be utilized for selecting the EMAT by selecting the EMAT with the highest S1 amplitude, the highest peak frequency based on scalogram, or the like. Also, upon detection of device migration, the methods of determining optimal EMAT may be repeated.

The frequency range and axis for detection is selected for determining the EMAT and additional HS signals can be obtained using that frequency range and axis. The HS signals and HS data obtained using the selected additional HS signals are more accurate and can be utilized for diagnosis of patient conditions, determining false determination, or the like. In one example, the additional HS signals are utilized at least as provided in US Patent Publication 2021/0350931 entitled METHOD AND SYSTEM FOR HEART CONDITION DETECTION USING AN ACCELEROMETER.

While Figure 9 provides an example method that is specific to determining the frequency range and axis to obtain HS signals that can be best used for EMAT determination using an ECG or EGM, the methodology of Figure 9 can be similarly used for other physiological determinations using an ECG or EGM. For example, the S2 feature of the HS signals can be utilized to identify time windows to search for the T wave timings in ECGs or EGMs (e.g., 100-0 msec preceding the occurrence of the S2 feature). T waves of an ECG or EGM can be found based on S2 detection. Often times, the T wave from an EGM may not be readily discernable or the signal amplitude or T wave may be too small to reliably detect. By using the methodology of Figure 9, the S2 detection can be obtained from the selected one of the six (6) bandwidth HS components to improve detection. By cycling through the 6 bandwidth HS components during a first period of time and selecting the bandwidth HS component that produces the best S2 signal to use during a second period of time, improved detection for the T wave can be accomplished.

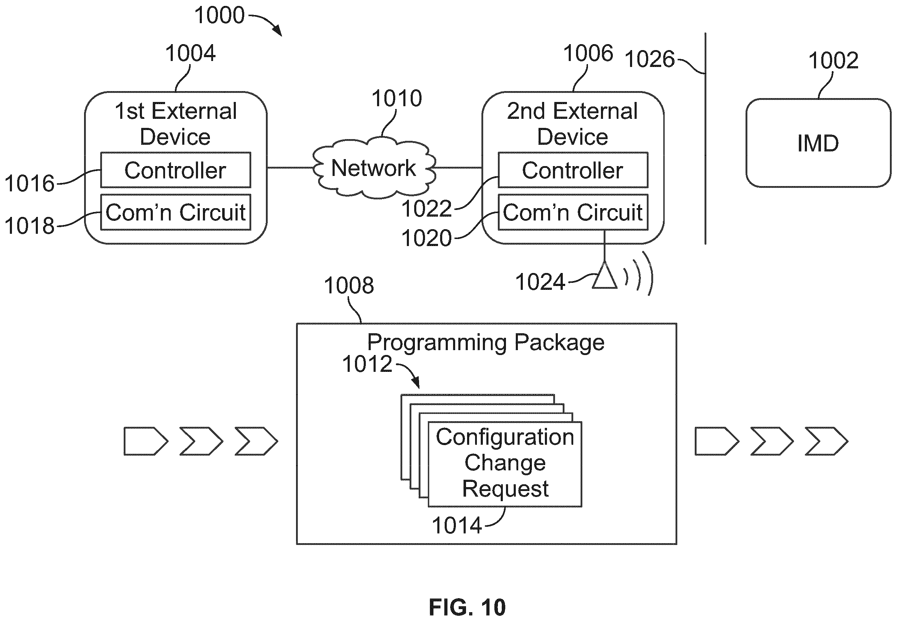

Figure 10 is a schematic illustration of a system 1000 according to an embodiment. In one example, the system 1000 is at least one of the system as illustrated and described in U.S. Pub. No. 2023/0414951, entitled SYSTEM AND METHOD FOR IMPLANTABLE MEDICAL DEVICE REMOTE PROGRAMMING. The system 1000 provides secure remote programming of an IMD 1002 while the IMD 1002 is implanted within a patient. In addition to the IMD 1002, the system 1000 includes one or more external devices. In the illustrated embodiment, the external devices include a first external device 1004 and a second external device 1006, but the system 1000 may include only a single external device or more than two external devices in other embodiments. The system 1000 may systems, devices, sensors, etc. previously described herein. For example, the IMD 1002 may represent the IMD 100 and the first external device 1004 may represent the external device 154.

The one or more external devices build a programming package 1008 that is designed to update an operating configuration of a programming session of the IMD 1002. The operating configuration of the programming session of the IMD 1002 generally refers to the specific settings, functions, parameters, and the like that dictate the device behavior. For example, the operating configuration affects how the IMD 1002 collects data, such as cardiac signals, how the IMD 1002 analyzes the data, and how the IMD 1002 responds to the data. The response may include activating the pulse generator or shocking circuit to deliver stimulation therapy to the patient, saving data in the memory device, communicating data to the external device 1006, or the like. Even if the IMD 1002 has been previously programmed, the operating configuration of the programming session may be updated over time due to physiological changes in the patient, unplanned movement of the IMD 1002 within the patient, or even to enhance functionality of the IMD 1002 based on software developments.

In the illustrated embodiment, the first external device 1004 builds the programming package 1008 related to the programming session. In a non-limiting example, the first external device 1004 is accessed and utilized by a clinician to select a collection 1012 of multiple configuration change requests 1014 for inclusion in the programming package 1008. The first external device 1004 may be a clinician programming device, a workstation, or a computing device (e.g., smartphone, tablet computer, laptop computer, etc.). The clinician may select the collection 1012 by communicating via a network 1010 with a server or other computing device in the cloud. For example, the clinician may be required log in to a secure portal prior to receiving access to select the configuration change requests 1014 and command the generation of the programming package 1008. The first external device 1004 may be remote from the second external device 1006 and the patient.

The first external device 1004 conveys the programming package 1008, as a message, to the second external device 1006 via the network 1010. In a non-limiting example, the second external device 1006 is a device that is disposed proximate to the patient, at least at a time that the second external device 1006 communicates the programming package 1008 to the IMD 1002. For example, the second external device 1004 may be a bedside monitoring device or a computing device utilized or possessed by the patient, such as a smartphone, tablet computer, laptop computer, or the like.

When the second external device 1006 receives the programming package 1008, the programming package 1008 may schedule a designated time in the future at which to transmit the programming package 1008 to the IMD 1002. The second external device 1006 initially stores the programming package 1008 in a memory device of the device 1006 until the designated time. At the designated time, the second external device 1006 wirelessly transmits the programming package 1008 to the IMD 1002 to update a programming session. This process of scheduling in advance the delivery of the programming package to the IMD via an intermediary device is referred to as asynchronous remote programming. The IMD 1002 is implanted within the patient at the time that the IMD 1002 receives the programming package 1008. According to embodiments herein, the IMD 1002 utilizes a virtual device engine (VDE) to simulate operation of the IMD based on the programming package. The VDE communicates with the IMD to ensure the most recent programming configuration is known. In an example, the VDE can verify whether the programming package is configured and based on the most recent and most updated programming session at the IMD. If all the elements of the programming package 1008 are verified, the IMD 1002 executes all the configuration change requests 1014 to update the operating configuration of the IMD 1002. On the other hand, if at least one of the elements are not verified, as described herein, the IMD 1002 does not execute any of the configuration change requests 1014, such that the operating configuration of the IMD 1002 is not updated based on the change request.

The first and second external devices 1004, 1006 include respective controller circuits (e.g., controllers) and communication circuits. Each of the controllers includes one or more processors. In the process described above, the controller 1016 of the first external device 1004 generates the programming package 1008. The communication circuit 1018 of the first external device 1004 communicates the programming package 1008 to the second external device 1006 via the network 1010. The communication circuit 1020 of the second external device 1006 receives the programming package 1008 and conveys the package 1008 to the controller 1022 of the second external device 1006 for analysis. The controller 1022 determines the scheduled delivery time and transmission characteristics, such as frequency channel and protocol, for communicating the programming package 1008 to the IMD 1002. At the scheduled delivery time, the controller 1022 utilizes the communication circuit 1020 to transmit the programming package 1008 to the IMD 1002. The communication circuit 1020 may include an antenna 1024 that wirelessly transmits the programming package 1008 via RF signals, such as Bluetooth. The signals that represent the programming package 1008 penetrate the body 1026 of the patient and reach a receiver of the IMD 1002. The receiver may be a communication modem, which conveys the received data packets to the one or more processors of the microcontroller for analysis. In this way, the IMD 1002 is beneficially able to receive the programming package 1008 without requiring a network connection or a proximity sensor.

In instances as described above in which the communication of the programming package 1008 from the source to the IMD 1002 is delayed and/or indirect, there is a risk that the contents of the programming package 1008 may be tampered with or corrupted after generation and before receipt by the IMD 1002. The system 1000 and method described herein perform cryptographic operations to ensure that the configuration change requests 1014 are immutable and that the source of the package 1008 is authenticated prior to execution by the IMD 1002.

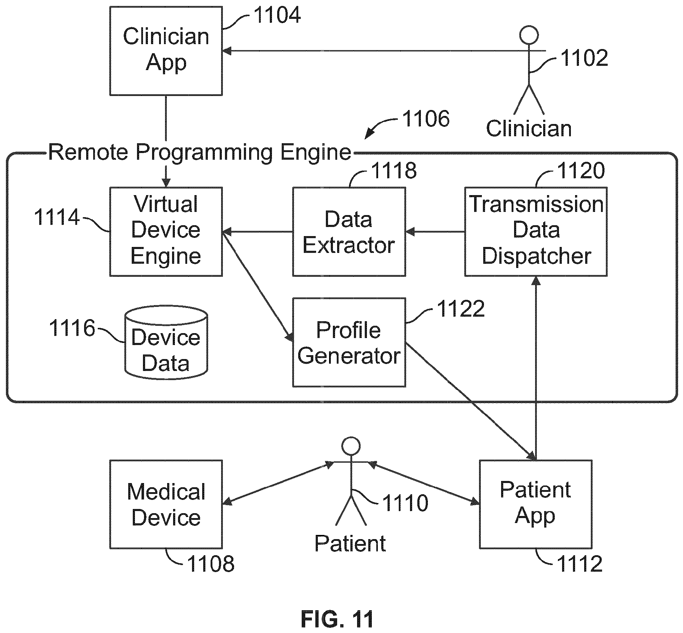

Figure 11 illustrates a system 1100 for verifying an update sent by a clinician 1102 from a clinician application 1104 through an RPE 1106 to an IMD 1108 of a patient 1110 that utilizes a patient application 1112. The patient application 1112 in one example can be accessed and programmed on a patient programming device. The update may be an update of a treatment, software, firmware, application, program, etc. The clinician application 1104 may be stored in a storage device of a clinician programming device, including a clinician laptop computer, desktop computer, CPU, smartphone, smartwatch, tablet, or the like. The clinician application 1104 may also be stored within a network, such as in the cloud, at a server, at a network resource, or the like. In each instance, the clinician application includes instructions that may be executed by one or more processors. The one or more processors can be of the clinician laptop computer, desktop computer, CPU, smartphone, smartwatch, tablet, etc., of a server, of a network device, or the like. Based on the instructions, the steps of a method or process for updating the IMD are provided.

The RPE 1106 may be in the cloud, in a network, at a server, over Bluetooth (BLE), or the like. The RPE 1106 includes a VDE 1114 that can simulate the operation of the IMD 1108. By simulating the operation of the IMD 1108, the VDE 1114 obtains information related to the IMD 1108, including all updates, changes, modifications, etc. that occur at the IMD 1108 over time. The VDE 1114 can store this IMD 1108 operational information at a device data store 1116. In one example, the RPE 1106 includes a data extractor 1118 that extracts such information from a transmission data dispatcher 1120 that continuously receives transmission data from the IMD 1108 via the patient application 1112.

By continuously obtaining the IMD data, the VDE 1114 can continuously simulate the operation and device activities of the IMD 1108, and store data at the device data store 1116. The VDE can thus manage a communication session, including a programming session, implemented by the clinician 1102 from initialization, to pending, to completed. In one example, when the clinician app communicates the desired modifications, changes, updates, etc. to the RPE 1106, the RPE can synchronize the current state of the IMD 1108 with the modification, change, update, etc. desired to be implemented. The VDE 1114 can provide the synchronized data and information to an asynchronous profile generator 1122 to generate an asynchronous remote programming profile that includes updated program instructions for updating the IMD 1108. In another example, if the IMD 1108 has been modified previously without the knowledge of the clinician 1102, the VDE 1114 ensures the desired update occurs on the most recent version of a program, software, firmware, application, or the like.

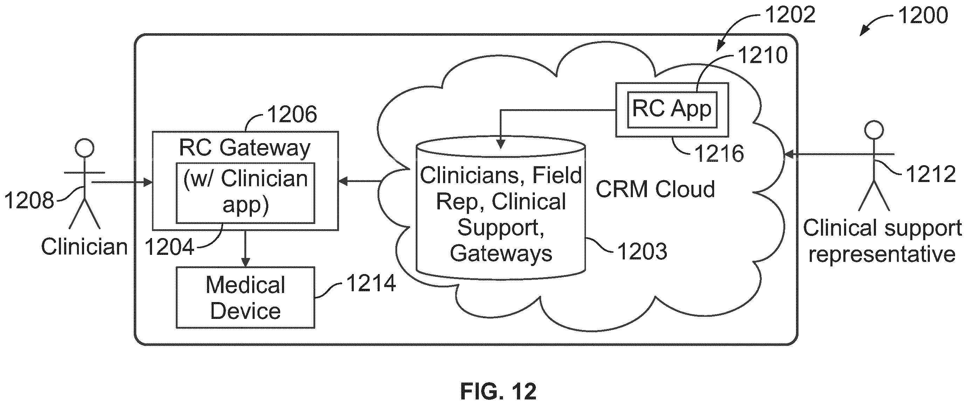

Figure 12 illustrates a medical device remote controlled (MDRC) system 1200 that includes an engine 1202, patient database 1203, a clinician application 1204 that can be accessed at a clinician electronic device 1206 (e.g., gateway) for local users 1208 (e.g., clinicians), an RC application 1210 at a remote electronic device 1216 (e.g., gateway) for RC users 1212 (e.g., clinical support representatives), and at least one medical device 1214. By providing the MDRC system 1200, communication sessions can be provided.

The engine 1202 can include one or more processors for executing instructions to perform processes and methods described herein. The engine 1202 can also include a memory or storage device for storing information, including patient information, medical device information, treatment information, etc. In an example, the engine 1202 is within a cloud environment that can be accessed by numerous electronic devices other than the clinician electronic device 1206 and the remote electronic device 1216. In one example, the engine 1202 is coupled to the patient database 1203 that includes patient information, clinician information, field representative information, clinical support information, medical device information, electronic device information, including electronic device information related to the clinician electronic device 1206 and the remote electronic device 1216, or the like. The information stored can be related to an individual patient, numerous patients, patient medical devices, generic medical devices, studies, tests, other patient information, etc. In all, the patient database 1203 and engine 1202 can be coupled within a network, including a mesh network, cloud network, Bluetooth network, etc. and communicate with plural clinician electronic devices, plural remote electronic devices, etc. to obtain information related to plural patients, medical devices, treatments, or the like. The patient database can include algorithms, including artificial intelligence algorithms for analyzing information obtained from the plural patients, medical devices, treatments, etc. to provide recommendations, modifications, updates, changes, or the like to medical devices, treatments, etc. accordingly.

The clinician electronic device 1206 is the electronic device that is at the location of the patient, and/or medical device 1214. The clinician electronic device 1206 is considered local because the patient, and/or medical device 1214 is at the location of the clinician electronic device 1206 and is not remote from the patient and/or medical device 1214. In one example the medical device 1214 is an IMD. In another example, the medical device may be a neurostimulation device, monitor, or the like. The clinician electronic device can be a laptop computer, desktop computer, mobile device, server, iPad, or the like.

The clinician electronic device 1206 can include one or more processors for executing instructions to perform processes and methods described herein. The clinician electronic device can also include a memory or storage device for storing information, including patient information, medical device information, treatment information, etc. The clinician electronic device can also include an interface so that a local user 1208, such as a clinician, can input information into the clinician electronic device 1206. The interface in one example is an input device that can include a touch screen, keyboard, mouse, microphone, or the like to provide information, commands, etc. to the clinician electronic device 1206. In addition, the clinician electronic device 1206 can include an output device, such as a screen, display, speakers, etc. for providing information and data to the local user 1208. The clinician electronic device 1206 can thus be utilized by the local user 1208 to obtain information directly from the medical device 1214, and/or a patient that has the medical device 1214.

When a local user turns on the clinician electronic device 1206, the clinician electronic device 1206 detects network connectivity and automatically performs an analysis to ensure the engine 1202 is operational. In addition, the analysis can determine the identification, name, serial number, etc. associated with the clinician electronic device 1206, the version of the software, firmware, or the like of the clinician application 1204, operating system, etc. of the clinician electronic device 1206, the location or region of the clinician electronic device 1206, or the like.

In addition, the remote electronic device 1216 is located remotely, or away from the patient and/or medical device 1214. The remote electronic device 1216 can be in a hospital, clinician's office, clinician's home, or the like that is not in the location of the patient and/or medical device 1214. The remote electronic device 1216 can be a laptop computer, desktop computer, mobile device, server, iPad, or the like that includes an interface so that a RC user 1208, such as a clinician support representative, can input information into the remote electronic device 1216. The remote electronic device 1216 in one example is an electronic device of a network.

The remote electronic device 1216 can include one or more processors and a memory for executing instructions and storing information like the clinician electronic device 1206 and engine 1202. In addition, the remote electronic device 1216, like the clinician electronic device 1206, can include an interface that is an input device, along with an output device. The remote electronic device 1216 also includes the RC application 1210 that includes program instructions for obtaining patient information from the patient database 1203 via the engine 1202. The RC applicant 1210 can also communicate with the clinician application 1204 to obtain physiological information related to a patient that is obtained from the medical device 1214 via the engine 1202.

When a RC user 1208 turns on the remote electronic device 1216, the remote electronic device 1216 detects network connectivity and automatically performs an analysis to ensure the engine 1202 is operational. In addition, the analysis can determine the identification, name, serial number, etc. associated with the remote electronic device 1216, the version of the software, firmware, or the like of the RC application 1210, operating system, etc. of the remote electronic device 1216, the location or region of the remote electronic device 1216, or the like.

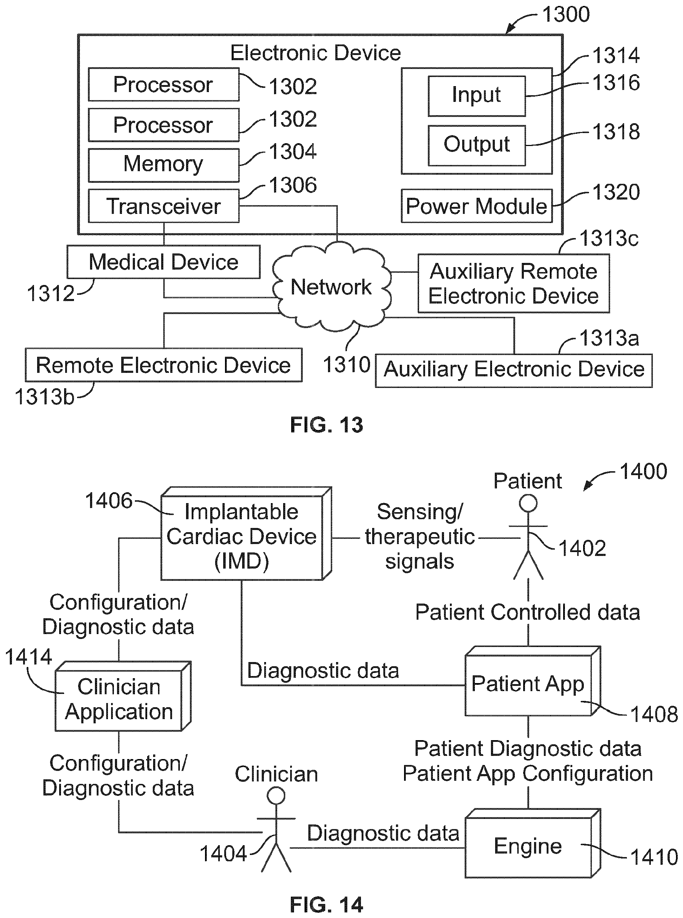

Fig. 13 illustrates a schematic block diagram of an electronic device 1300. In one example the electronic device 1300 is the clinician electronic device of Fig. 12, whereas in another example the electronic device 1300 can be the remote electronic device of Fig. 12. The electronic device 1300 can be a laptop computer, desktop computer, mobile device, server, iPad, or the like. The electronic device 1300 can include one or more processors 1302, a storage device or memory 1304, and a transceiver 1306. The transceiver 1306 is configured to communicate with other electronic devices through a network 1310. The transceiver can communicate with a server, engine, or the like, located in the cloud, over Bluetooth, etc. In addition, the transceiver can communicate with a medical device 1312. The transceiver can communicate with the medical device 1312 remotely through the network 1310, or directly. In another example, the transceiver 1306 can communicate with an auxiliary electronic device 1313a, a remote electronic device 1313b, or a remote auxiliary electronic device 1313c. In example embodiments, the auxiliary electronic device 1313a can be a smartphone, Ipad, or the like that can receive short message service (SMS) messages, such as text messages over the network. The remote auxiliary electronic device 1313c similarly can be a smartphone, Ipad, or the like that can also receive SMS messages. In one example, the system can receive identification information from a clinician, clinician support representative, etc. and an SMS message can be provided by the system to the auxiliary electronic device 1313a and/or 1313c to provide verification that the clinician, or clinician support representative indeed provided the identification information. The identification information can be a username, password, login, or the like. By using a communication signal between the auxiliary electronic device 1313a and remote auxiliary device 1313c, additional security is provided.

The electronic device 1300 can also include an interface 1314 that can include an input 1316 such as a touchscreen, keyboard, mouse, microphone, etc. The electronic device 1300 can also include an output 1318 that can also include a touchscreen, display, speakers, etc. In one example, the input 1316 and output 1318 can include the same interface, display, etc. The electronic device 1300 also includes a power module 1320 for powering the electronic device 1300.

Figure 14 illustrates yet another example MDRC system 1400 that can be utilized for remote care of a patient 1402 by a clinician 1404. In one example, the patient 1402 ensures connectivity between components of the MDRC system (e.g., the IMD, patient application, and engine). For example, the patient 1402 ensures a patient device such as a smart phone is charged and turned on with mobile data and access to the internet also functioning. The patient also stays within proximity of the patient device during the duration of the data collection.

In this embodiment, the patient has the IMD 1406 and can have a patient device that may have a patient application 1408 that is in communication with the IMD 1406. The IMD 1406 can be any medical device such as Implantable Loop Recorder, Pacemaker, ICD or CRT with an incorporated 3D AXL. When a HS feature is enabled, the IMD can obtain and processes the HS data from the 3D AXL, save the HS data in a device memory, or makes the HS data available for retrieval by an external instrument such as the patient device via the patient application 1408. The patient application 1408 implements the aspect of a HS data manager that executes workflow schedules and associated operations on the IMD 1406 to enable/disable the HS feature in the IMD 1406, retrieve HS data and reset HS data collection sessions.

The patient application 1408 can be in communication with an engine 1410 that may include a database, processing software and hardware, or the like. The engine 1410 can implement aspects of a HS data manager that in one example can generate patient application schedules and associated operations for the HS workflows based on settings selected by a user. In one example, the user may be the patient, whereas alternatively, the use can be a local clinician. The engine 1410 can also implement a storage object when the engine 1410 receives and stores uploaded HS data. HS data can be displayed directly to a remote clinician 1404 on the engine 1410 via a clinician application 1414 and can be used for further processing with different HS algorithms.

The clinician application 1414 may retrieve and store HS data as part of a session record during a normal in-clinic follow-up. Data retrieval by the clinician application 1414 is not the primary means of collecting the HS data. As such, the data collected by the clinician application 1414 should remain available for retrieval by the patient application 1408.

When the MDRC system 1400 is in use the IMD 1406 implements the collection of heart sounds data from a 3-D AXL and makes the data available for retrieval by an external instrument such as a patient device (e.g., patient electronic device). This process can be referred to as IMD HS data collection, to distinguish from the process of reading the HS data from the IMD and uploading the HS data the engine 1410 for storage.

The 3-D AXL can support the following modes: standby, single-axis (1D activity), Normal (3D activity/posture), and HS. In one example, each mode is exclusive (only one mode can occur at a time). The 3D AXL in HS mode allows for the heart sound data to be measured in 3 axes (x,y,z) for each of the two available filter settings, wideband (WB) and narrowband (NB), for a total of 6 bandwidth HS components (x-NB, y-NB, z-NB, x-WB, y-WB, z-WB) as Fig. 3A illustrates. The data has to be read sequentially for each axis and filter combination since the sensor does not support multiple simultaneous bandwidth HS components.

To provide distribution and sampling of HS data (e.g., across different heart rates), the IMD HS data collection can be split into several separate collection opportunities rather than collecting a long duration of HS data all at one time. In one example, the device can obtain HS data during discrete HS data collection sessions. There can be either a fixed or a programmable number of sessions, separated by some amount of time per retrieval cycle. The IMD data collection cycle is synchronized to retrieval of the data and reset of the collection by the patient application 1408.

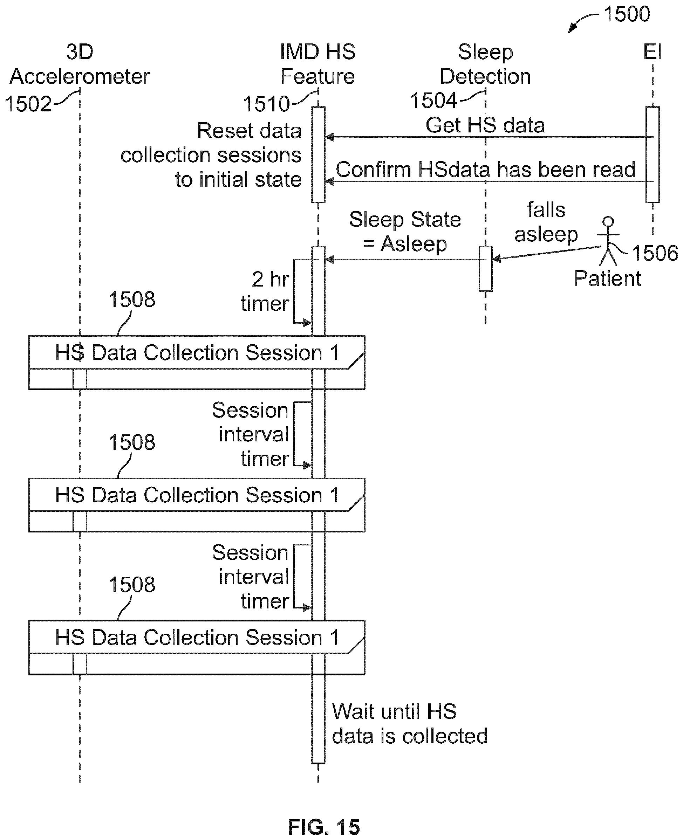

Fig. 15 illustrates a schematic diagram of a process 1500 for obtaining HS data from a 3-D AXL 1502. Due to the noisy nature of the HS signal the patient should be at rest (not moving) during the data collection. To determine when the patient is at rest, the HS feature may use a sleep detection feature 1504. The sleep detection feature 1504 uses 1D activity and trended posture measurements to determine if the patient is in low activity, recline or lying down state. In one example, the sleep detection feature is a processor that continuously obtains AXL data related to the movement, posture, or otherwise related to a patient and analyzes the AXL data to determine that a patient 1506 has been laying down for a determined period of time. The determination that the patient 1506 is laying down can be as a result of comparing the AXL data to previously obtained AXL data of the AXL or another AXL in another patient, used in an algorithm, or the like. In one example, a 1-D activity algorithm can be utilized for a determined period, such as for 15 days, to be trained on the sleeping habits of each specific patient. The AXL data obtained during the determined period can be utilized for comparison, or forming an algorithm, to determine that a patient 1506 is sleeping. In an example, the sleep detection feature 1504 can provide an indication of sleep entry and sleep exit (e.g., when the patient is awake).

In one example, to provide additional confidence that the patient 1506 is in a steady state, the first HS data collection can be provided over a determined period, such as 2 hours (this value can be programmable), after sleep entry. The determined period is thus considered a HS data collection session 1508 during which HS data is obtained by an IMD 1510. The time between sessions can be an adjustable value, nominally also a determined period such as 2 hours, contingent no sleep exit occurs between the sessions 1508. If the IMD determines that that awakes during a session 1508, the IMD can stop obtaining data and discard the HS data for the interrupted session. In one example, the AXL data obtained indicates that a patient 1506 has moved from a prone position to a sitting up, or standing position in a manner like which the laying down position can be determined. The IMD 1510 can continue to monitor the AXL data to determine when a patient 1506 is once again laying down to begin a new session 1508.

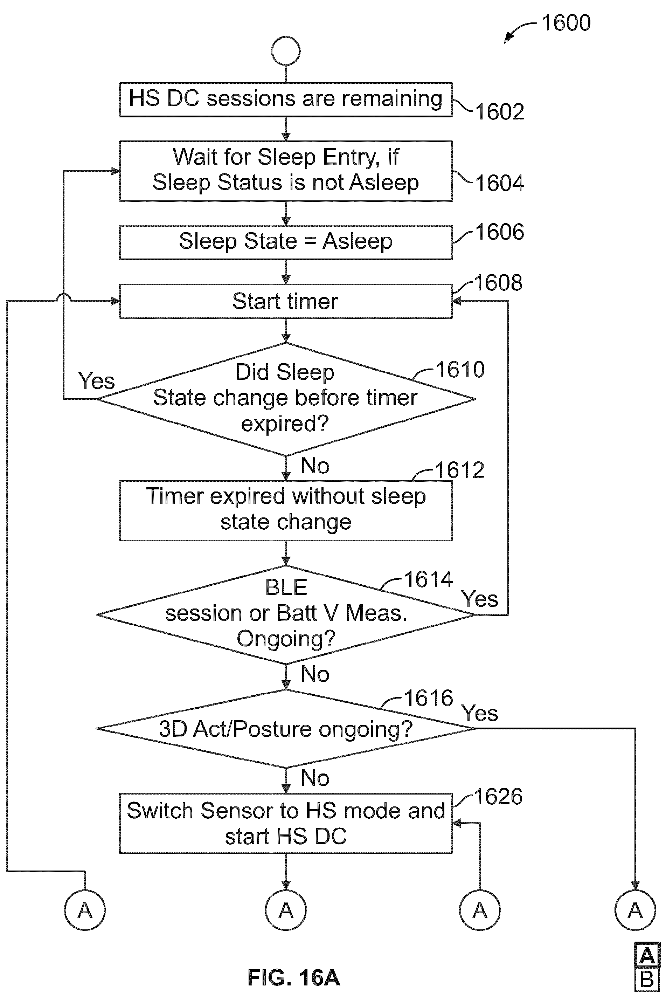

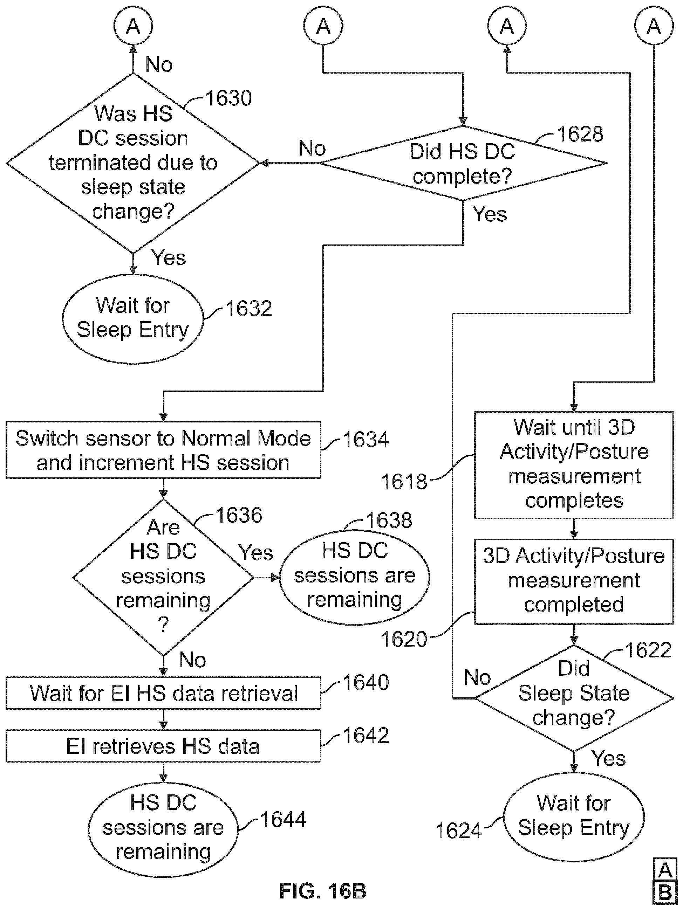

Figure 16 is a schematic block flow diagram of a process 1600 for obtaining HS data. In one example, the systems and devices of Figures 1-7 and 15 are utilized to implement the steps of the process 1600. At 1602 one or more processors monitor a patient to determine if a patient is sleeping. In one example, the one or more processors continuously obtain AXL data to determine the position, posture, and/or activity of the patient. In another example 1D activity and trended posture measurements are utilized to make the determination.

At 1604, the one or more processors determine that a patient has entered a sleep state. In one example, the obtained AXL data is compared to previously obtained AXL data to determine that a patient is laying down, is not moving, or the like. In another example, an algorithm may be utilized to make such a determination. In yet another example, a mathematical model, function, or the like can be used to make the determination that the patient has entered a sleep state. At 1606, the one or more processors determine the patient is in a sleep state.

At 1608, the one or more processors in response to determining that the patient has entered a sleep state, begins a timer for starting a session during which the one or more processors obtain HS data. The timer can be for a determined period such as a half hour, one hour, two hours, twelve hours, etc. The duration of the time can dynamically change for each session depending on characteristics of interest related to the patient. Alternatively, the timer can be static and the same for each session unless a manual input is provided to vary the determined period.

At 1610, the one or more processors determine whether the sleep state of the patient has changed while the session (e.g., during the determined period) is occurring. In an example, a timer is provided to determine the determined period of time, and the one or more processors continue to obtain AXL data that indicates that the patient has changed positions, that patient activity is occurring, or the like. In another example, the one or more processors communicate with sensors, other medical devices, etc. that can be utilized to determine that a patient is awake. Regardless, a determination is made if a sleep state has been interrupted before the determined period of the timer has ended. If the sleep state has been interrupted, the one or more processors can determine whether the patient goes back into a sleep state.

Alternatively, if at 1610 the sleep state is determined not to be interrupted, at 1612 the timer expires without the sleep state being interrupted. At 1614 the one or more processors determine whether additional measurements of any kind are still ongoing. In one example, a check is made to determine whether any other program is in use and attempting to obtain patient data that would require HS data, other AXL data, etc. while the patient is sleeping. If yes, the timer is reset for another determined period during which readings continue to be obtained. Alternatively, if no additional readings are to be obtained, at 1616, the one or more processors determine whether activity data or posture data is being obtained. A check can be made to ensure that a request for additional AXL data has not been requested. If additional activity data and posture data is being obtained, at 1618 and 1620 the one or more processors continue obtaining the activity and posture data until such obtaining is complete. Once complete, at 1622, the one or more processors determine if a sleep state has changed. If not, at 1624, the one or more processors wait until sleep entry.

If at 1616 the one or more processors determine activity and posture data is not being obtained, or if at 1622 the one or more processors determine that the sleep state has changed, at 1626 the one or more processors switch the AXL/sensor to HS mode to begin obtaining AXL data. At 1628, the one or more processors continuously monitor determine if the HS mode is done obtaining the AXL data. If not, at 1630, the one or more processors determine if a change in sleep state interrupted the AXL data acquisition. If not, the timer at 1608 can be restarted; however, if sleep state did terminate, at 1632 the one or more processors again wait for sleep entry.

If at 1628 the one or more processors determine that all the AXL data desired has been obtained, at 1634, the AXL/sensor is switched to a normal mode for increment HS sessions. At 1636, the one or more processors determine whether additional data collection session(s) are remaining. If so, at 1638 the one or more processors conduct the additional session(s) to collect HS data. If at 1636 no more sessions are present, at 1640, the one or more processors wait for HS data retrieval. Once retrieved at 1642, at 1644 the one or more processors conduct remaining sessions to obtain HS data.

In all, the IMD can store HS data during each HS data collection sessions including ensemble average of multiple R-wave-aligned heart sounds collected over a collection period for each HS sensor configuration. The alignment of R-waves can be either from the start of the QRS complex or from the peak of the R-wave. Addition HS data collected can include raw heart sound waveform snippets for each bandwidth HS component taken from the same measurement as the ensemble average, with an indication of position of the sensed event. Because the HS waveforms are different in each of the HS sensor configurations (see Figure 3A), each bandwidth HS component needs a separate ensemble average. Additionally, each data collection session needs to have its own set of ensemble averages, rather than adding on to the previously collected data, since the heart rate or patient position may differ from session to session, possibly changing the morphology or timing of heart sounds. A histogram of the distribution of the intervals used in calculating each ensemble average is useful in gauging the quality of the ensemble average and the underlying HR.

Embodiments may be implemented in connection with one or more IMDs. Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker, and the like. The IMD may measure electrical and/or mechanical information. For example, the IMD may include one or more structural and/or functional aspects of the device(s) described in U.S. Patent Number 9,333,351, entitled NEUROSTIMULATION METHOD AND SYSTEM TO TREAT APNEA, and U.S. Patent Number 9,044,610, entitled SYSTEM AND METHODS FOR PROVIDING A DISTRIBUTED VIRTUAL STIMULATION CATHODE FOR USE WITH AN IMPLANTABLE NEUROSTIMULATION SYSTEM. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled LEADLESS IMPLANTABLE MEDICAL DEVICE HAVING REMOVABLE AND FIXED COMPONENTS, and U.S. Patent Number 8,831,747, entitled LEADLESS NEUROSTIMULATION DEVICE AND METHOD INCLUDING THE SAME. Additionally or alternatively, the IMD may include one or more structural and/or functional aspects of the device(s) described in U.S. Patent Number 8,391,980, entitled METHOD AND SYSTEM FOR IDENTIFYING A POTENTIAL LEAD FAILURE IN AN IMPLANTABLE MEDICAL DEVICE, and U.S. Patent Number 9,232,485, entitled SYSTEM AND METHOD FOR SELECTIVELY COMMUNICATING WITH AN IMPLANTABLE MEDICAL DEVICE. Additionally or alternatively, the IMD may be a subcutaneous IMD that includes one or more structural and/or functional aspects of the device(s) described in U.S. Patent Number 10,765,860, entitled SUBCUTANEOUS IMPLANTATION MEDICAL DEVICE WITH MULTIPLE PARASTERNAL-ANTERIOR ELECTRODES; U.S. Patent Number 10,722,704, entitled IMPLANTABLE MEDICAL SYSTEMS AND METHODS INCLUDING PULSE GENERATORS AND LEADS; U.S. Patent Number 11,045,643, entitled SINGLE SITE IMPLANTATION METHODS FOR MEDICAL DEVICES HAVING MULTIPLE LEADS. Further, one or more combinations of IMDs may be utilized from the above mentioned patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled IMPLANTABLE MEDICAL SYSTEMS AND METHODS INCLUDING PULSE GENERATORS AND LEADS; U.S. Patent Number 10,765,860, entitled SUBCUTANEOUS IMPLANTATION MEDICAL DEVICE WITH MULTIPLE PARASTERNAL-ANTERIOR ELECTRODES. The IMD may represent a passive device that utilizes an external power source and/or an active device that includes an internal power source. The IMD may deliver some type of therapy/treatment, provide mechanical circulatory support and/or merely monitor one or more physiologic characteristics of interest (e.g., PAP, CA signals, impedance, heart sounds). Additionally or alternatively, embodiments may be implemented in connection with one or more passive IMDS (PIMDs). Non-limiting examples of PIMDs may include passive wireless sensors used by themselves or incorporated into or used in conjunction with other IMDs, such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, embodiments may implement one or more structural and/or functional aspects of the device(s) described in U.S. Patent No. 9,265,428 entitled IMPLANTABLE WIRELESS SENSOR, U.S. Patent No. 8,278,941 entitled STRAIN MONITORING SYSTEM AND APPARATUS, U.S. Patent No. 8,026,729 entitled SYSTEM AND APPARATUS FOR IN-VIVO ASSESSMENT OF RELATIVE POSITION OF AN IMPLANT, U.S. Patent No. 8,870,787 entitled VENTRICULAR SHUNT SYSTEM AND METHOD, and U.S. Patent No. 9,653,926 entitled PHYSICAL PROPERTY SENSOR AND ACTIVE ELECTRONIC CIRCUIT AND WIERELESS POWER AND DATA TRANSMISSION.

Additionally or alternatively, embodiments herein may be implemented in connection with the methods and systems described in METHOD AND DEVICE FOR DETECTING RESPIRATION ANOMALY FROM LOW FREQUENCY COMPONENT OF ELECTRICAL CARDIAC ACTIVITY SIGNALS, U.S. Pub. No. 2021/0345891.

Additionally or alternatively, embodiments herein may be implemented in connection with the methods and systems described in SYSTEM FOR VERIFYING A PATHOLOGIC EPISODE USING AN ACCELEROMETER, Patent Number 11,980,472.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described, and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices, and program products according to various example embodiments. These program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processorbased or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. An implantable medical device, IMD, (100), comprising:
a heart sound, HS, sensor (270) configured to sense HS signals along an axis over a first period of time;
a filtering assembly (404) configured to filter the HS signals utilizing first and second bandwidths to output first and second bandwidth HS components;
a memory (260) to store specific executable instructions; and
one or more processors (220) that, when executing the specific executable instructions, are configured to:
identify a first characteristic of interest, COI, of a heartbeat from the first bandwidth HS component;
identify a second COI of the heartbeat from the second bandwidth HS component, wherein the second COI is a same COI of a heartbeat as the first COI;
select one of the first and second bandwidths based on a comparison of the first and second COI;
obtain additional HS signals during a second period of time; and
utilize the one of the first and second bandwidths selected to filter the additional HS signals.

2. The IMD of claim 1, wherein the first period of time includes first and second intervals, the first bandwidth HS component sensed during the first interval, the second bandwidth HS component sensed during the second interval.

3. The IMD of claim 1 or 2, wherein the first bandwidth has a different frequency range than the second bandwidth.

4. The IMD of any one of claims 1 to 3, wherein the additional HS signals are not filtered utilizing the one of the first and second bandwidths that is not selected.

5. The IMD of any one of claims 1 to 4, wherein the one or more processors (220) are further configured to determine a first electromechanical activation time, EMAT, based on the first COI of the heartbeat.

6. The IMD of any one of claims 1 to 5, wherein the axis is a first axis and the HS sensor (270) includes a second axis and a third axis, and the one or more processors (220) are further configured to:
identify a third COI of the heartbeat from a third bandwidth component related to the second axis;
identify a fourth COI of the heartbeat from a fourth bandwidth component related to the second axis;
select one of the first, second, third, or fourth bandwidth components based on a comparison of the first, second, third, and fourth COI; and
utilize the one of the first, second, third or fourth bandwidth components selected to filter the additional HS signals.

7. The IMD of any one of claims 1 to 6, wherein the one or more processors (220) are further configured to treat a physiologic condition based on the additional HS signals.

8. The IMD of any one of claims 1 to 7, wherein the first COI is a COI selected from the group consisting of S1 amplitude, S2 amplitude, S3 amplitude, S1 to S2 interval, S2 to S3 interval, and S3 to S1 interval and the second COI is the selected COI.

9. The IMD of any one of claims 1 to 8, wherein the axis is a first axis and the HS sensor (270) includes a second axis and a third axis, and the one or more processors (220) are further configured to:
operate the IMD (100) in a standby mode wherein no HS signals are sensed;
operate the IMD (100) in a single-axis mode, wherein HS signals are only obtained from the first axis; and
operate in a three-axis mode, wherein HS signals are sequentially obtained from the first axis and then from the second axis.

10. The IMD of any one of claims 1 to 9, wherein the one or more processors (220) are further configured to:
obtain HS data from the additional HS signals; and
communicate the HS data to a first external device (1004).

11. The IMD of claim 10, wherein to obtain the HS data from the additional HS signals, the one or more processors (220) are further configured to:
determine that a patient is sleeping based on one-dimensional activity or trended posture measurements by comparing accelerometer data related to movement or posture of the patient to previously obtained accelerometer data representative of a sleeping patient; and
record the additional HS signals for a determined period in response to determining that the patient is sleeping.

12. The IMD of any one of claims 1 to 11, wherein to select one of the first and second bandwidths the one or more processors (220) are configured to determine a first ensemble average HS signal related to the first bandwidth HS component and determine a second ensemble average HS signal related to the second bandwidth HS and compare the first ensemble average HS signal and the second ensemble average HS signal.

13. The IMD of any one of claims 1 to 12, wherein the one or more processors (220) are further configured to:
determine a first electromechanical activation time, EMAT, based on the first COI of the heartbeat;
determine a second EMAT based on the second COI of the heartbeat;
select one of the first EMAT or the second EMAT to provide a selected EMAT based on a shared characteristic of the first EMAT and second EMAT; and
obtain the additional HS signals during the second period of time based on the selected EMAT.

14. The IMD of any one of claims 1 to 12, wherein the one or more processors (220) are further configured to:
determine a first electromechanical activation time, EMAT, based on the first bandwidth HS component;
determine a second EMAT based the second bandwidth HS component;
determine a third EMAT based on a third bandwidth HS component;
select one of the first EMAT, the second EMAT, or the third EMAT to provide a selected EMAT based on a shared characteristic of the first EMAT, second EMAT, and third EMAT; and
obtain the additional HS signals based on the selected EMAT.

15. The IMD of any one of claims 1 to 12, wherein the one or more processors (220) are further configured to:
determine a first electromechanical activation time, EMAT, based on the first bandwidth HS component and the first COI of the heart beat; determine at least one of 1) a second EMAT based on the second bandwidth HS component and the second COI of the heart beat or 2) a third EMAT based on a third bandwidth HS component and a third COI of the heart beat;
select one of the first EMAT, the second EMAT, or the third EMAT to provide a selected EMAT based on a shared characteristic of the first EMAT, second EMAT, and third EMAT; and
obtain the additional HS signals based on the selected EMAT.

## Patentansprüche

1. Implantierbare medizinische Vorrichtung, IMD, (100), umfassend:
einen Herztonsensor, HS-Sensor, (270), der dazu konfiguriert ist, HS-Signale entlang einer Achse über einen ersten Zeitraum zu erfassen;
eine Filterbaugruppe (404), die dazu konfiguriert ist, die HS-Signale unter Verwendung einer ersten und einer zweiten Bandbreite zu filtern, um eine HS-Komponente erster und eine zweiter Bandbreite auszugeben;
einen Speicher (260), um konkrete ausführbare Anweisungen zu speichern; und
einen oder mehrere Prozessoren (220), die, wenn sie die konkreten ausführbaren Anweisungen ausführen, zu Folgendem konfiguriert sind:
Identifizieren eines ersten interessierenden
Charakteristikums, COI, eines Herzschlags aus der HS-Komponente erster Bandbreite;
Identifizieren eines zweiten COI des Herzschlags aus der HS-Komponente zweiter Bandbreite, wobei das zweite COI ein gleiches COI eines Herzschlags wie das erste COI ist;
Auswählen einer der ersten und der zweiten Bandbreite auf der Basis eines Vergleichs des ersten und des zweiten COI;
Erlangen zusätzlicher HS-Signale während eines zweiten Zeitraums; und
Verwenden der einen der ersten und der zweiten Bandbreite, die ausgewählt ist, um die zusätzlichen HS-Signale zu filtern.

2. IMD nach Anspruch 1, wobei der erste Zeitraum ein erstes und ein zweites Intervall beinhaltet, wobei die HS-Komponente erster Bandbreite während des ersten Intervalls erfasst wird, wobei die HS-Komponente zweiter Bandbreite während des zweiten Intervalls erfasst wird.

3. IMD nach Anspruch 1 oder 2, wobei die erste Bandbreite einen unterschiedlichen Frequenzbereich zu der zweiten Bandbreite aufweist.

4. IMD nach einem der Ansprüche 1 bis 3, wobei die zusätzlichen HS-Signale nicht unter Verwendung der einen der ersten und der zweiten Bandbreite gefiltert werden, die nicht ausgewählt ist.

5. IMD nach einem der Ansprüche 1 bis 4, wobei der eine oder die mehreren Prozessoren (220) ferner dazu konfiguriert sind, einen ersten elektromechanischen Aktivierungszeitpunkt, EMAT, auf der Basis des ersten COI des Herzschlags zu bestimmen.

6. IMD nach einem der Ansprüche 1 bis 5, wobei die Achse eine erste Achse ist und der HS-Sensor (270) eine zweite Achse und eine dritte Achse beinhaltet und der eine oder die mehreren Prozessoren (220) ferner zu Folgendem konfiguriert sind:
Identifizieren eines dritten COI des Herzschlags aus einer Komponente dritter Bandbreite, die sich auf die zweite Achse bezieht;
Identifizieren eines vierten COI des Herzschlags aus einer Komponente vierter Bandbreite, die sich auf die zweite Achse bezieht;
Auswählen einer der Komponente erster, zweiter, dritter oder vierter Bandbreite auf der Basis eines Vergleichs des ersten, des zweiten, des dritten und des vierten COI; und
Verwenden der einen der Komponente erster, zweiter, dritter oder vierter Bandbreite, die ausgewählt ist, um die zusätzlichen HS-Signale zu filtern.

7. IMD nach einem der Ansprüche 1 bis 6, wobei der eine oder die mehreren Prozessoren (220) ferner dazu konfiguriert sind, eine physiologische Erkrankung auf der Basis der zusätzlichen HS-Signale zu behandeln.

8. IMD nach einem der Ansprüche 1 bis 7, wobei das erste COI ein COI ist, das aus der Gruppe ausgewählt ist, die aus einer S1-Amplitude, einer S2-Amplitude, einer S3-Amplitude, einem S1-bis-S2-Intervall, einem S2-bis-S3-Intervall und einem S3-bis-S1-Intervall besteht, und das zweite COI das ausgewählte COI ist.

9. IMD nach einem der Ansprüche 1 bis 8, wobei die Achse eine erste Achse ist und der HS-Sensor (270) eine zweite Achse und eine dritte Achse beinhaltet und der eine oder die mehreren Prozessoren (220) ferner zu Folgendem konfiguriert sind:
Betreiben der IMD (100) in einem Bereitschaftsmodus, wobei keine HS-Signale erfasst werden;
Betreiben der IMD (100) in einem Einzelachsenmodus, wobei HS-Signale lediglich von der ersten Achse erlangt werden; und
Betreiben in einem Dreiachsenmodus, wobei HS-Signale sequenziell von der ersten Achse und sodann von der zweiten Achse erlangt werden.

10. IMD nach einem der Ansprüche 1 bis 9, wobei der eine oder die mehreren Prozessoren (220) ferner zu Folgendem konfiguriert sind:
Erlangen von HS-Daten aus den zusätzlichen HS-Signalen; und
Kommunizieren der HS-Daten an eine erste externe Vorrichtung (1004).

11. IMD nach Anspruch 10, wobei, um die HS-Daten aus den zusätzlichen HS-Signalen zu erlangen, der eine oder die mehreren Prozessoren (220) ferner zu Folgendem konfiguriert sind:
Bestimmen, dass ein Patient schläft, auf der Basis eindimensionaler Aktivität oder tendierter Haltungsmessungen durch Vergleichen von Beschleunigungsmesserdaten, die sich auf eine Bewegung oder eine Haltung des Patienten beziehen, mit im Vorfeld erlangten Beschleunigungsmesserdaten, die einen schlafenden Patienten darstellen; und
Aufzeichnen der zusätzlichen HS-Signale für einen bestimmten Zeitraum als Reaktion auf das Bestimmen, dass der Patient schläft.

12. IMD nach einem der Ansprüche 1 bis 11, wobei, um eine der ersten und der zweiten Bandbreite auszuwählen, der eine oder die mehreren Prozessoren (220) dazu konfiguriert sind, ein erstes Gesamtmittelwert-HS-Signal, das sich auf die HS-Komponente erster Bandbreite bezieht, zu bestimmen und ein zweites Gesamtmittelwert-HS-Signal, das sich auf die HS zweiter Bandbreite bezieht, zu bestimmen und das erste Gesamtmittelwert-HS-Signal und das zweite Gesamtmittelwert-HS-Signal zu vergleichen.

13. IMD nach einem der Ansprüche 1 bis 12, wobei der eine oder die mehreren Prozessoren (220) ferner zu Folgendem konfiguriert sind:
Bestimmen eines ersten elektromechanischen Aktivierungszeitpunkts, EMAT, auf der Basis des ersten COI des Herzschlags;
Bestimmen eines zweiten EMAT auf der Basis des zweiten COI des Herzschlags;
Auswählen eines des ersten EMAT oder des zweiten EMAT, um einen ausgewählten EMAT auf der Basis eines geteilten Charakteristikums des ersten EMAT und des zweiten EMAT bereitzustellen; und
Erlangen der zusätzlichen HS-Signale während des zweiten Zeitraums auf der Basis des ausgewählten EMAT.

14. IMD nach einem der Ansprüche 1 bis 12, wobei der eine oder die mehreren Prozessoren (220) ferner zu Folgendem konfiguriert sind:
Bestimmen eines ersten elektromechanischen Aktivierungszeitpunkts, EMAT, auf der Basis der HS-Komponente erster Bandbreite;
Bestimmen eines zweiten EMAT auf der Basis der HS-Komponente zweiter Bandbreite;
Bestimmen eines dritten EMAT auf der Basis einer HS-Komponente dritter Bandbreite;
Auswählen eines des ersten EMAT, des zweiten EMAT oder des dritten EMAT, um einen ausgewählten EMAT auf der Basis eines geteilten Charakteristikums des ersten EMAT, des zweiten EMAT und des dritten EMAT bereitzustellen; und
Erlangen der zusätzlichen HS-Signale auf der Basis des ausgewählten EMAT.

15. IMD nach einem der Ansprüche 1 bis 12, wobei der eine oder die mehreren Prozessoren (220) ferner zu Folgendem konfiguriert sind:
Bestimmen eines ersten elektromechanischen Aktivierungszeitpunkts, EMAT, auf der Basis der HS-Komponente erster Bandbreite und des ersten COI des Herzschlags;
Bestimmen mindestens eines 1) eines zweiten EMAT auf der Basis der HS-Komponente zweiter Bandbreite und des zweiten COI des Herzschlags oder 2) eines dritten EMAT auf der Basis einer HS-Komponente dritter Bandbreite und eines dritten COI des Herzschlags;
Auswählen eines des ersten EMAT, des zweiten EMAT oder des dritten EMAT, um einen ausgewählten EMAT auf der Basis eines geteilten Charakteristikums des ersten EMAT, des zweiten EMAT und des dritten EMAT bereitzustellen; und
Erlangen der zusätzlichen HS-Signale auf der Basis des ausgewählten EMAT.

## Revendications

1. Dispositif médical implantable, IMD, (100), comprenant :
un capteur de bruits du cœur, HS, (270) configuré pour détecter des signaux HS le long d'un axe sur une première période de temps ;
un ensemble de filtrage (404) configuré pour filtrer les signaux HS en utilisant des première et deuxième bandes passantes pour délivrer en sortie des première et deuxième composantes HS de bande passante ;
une mémoire (260) pour stocker des instructions exécutables spécifiques ; et
un ou plusieurs processeurs (220) qui, lors de l'exécution des instructions exécutables spécifiques, sont configurés pour :
identifier une première caractéristique d'intérêt, COI, d'un battement de cœur provenant de la première composante HS de bande passante ;
identifier une deuxième COI du battement de cœur provenant de la deuxième composante HS de bande passante, dans lequel la deuxième COI est la même COI d'un battement de cœur que la première COI ;
sélectionner l'une des première et deuxième bandes passantes sur la base d'une comparaison des première et deuxième COI ;
obtenir des signaux HS supplémentaires pendant une seconde période de temps ; et
utiliser l'une des première et deuxième bandes passantes sélectionnée pour filtrer les signaux HS supplémentaires.

2. IMD selon la revendication 1, dans lequel la première période de temps comprend des premier et second intervalles, la première composante HS de bande passante étant détectée pendant le premier intervalle, la deuxième composante HS de bande passante étant détectée pendant le second intervalle.

3. IMD selon la revendication 1 ou 2, dans lequel la première bande passante présente une plage de fréquences différente de celle de la deuxième bande passante.

4. IMD selon l'une quelconque des revendications 1 à 3, dans lequel les signaux HS supplémentaires ne sont pas filtrés en utilisant l'une des première et deuxième bandes passantes qui n'est pas sélectionnée.

5. IMD selon l'une quelconque des revendications 1 à 4, dans lequel les un ou plusieurs processeurs (220) sont en outre configurés pour déterminer un premier temps d'activation électromécanique, EMAT, sur la base de la première COI du battement de cœur.

6. IMD selon l'une quelconque des revendications 1 à 5, dans lequel l'axe est un premier axe et le capteur HS (270) comprend un deuxième axe et un troisième axe, et les un ou plusieurs processeurs (220) sont en outre configurés pour :
identifier une troisième COI du battement de cœur provenant d'une troisième composante de bande passante liée au deuxième axe ;
identifier une quatrième COI du battement de cœur provenant d'une quatrième composante de bande passante liée au deuxième axe ;
sélectionner l'une des première, deuxième, troisième ou quatrième composantes de bande passante sur la base d'une comparaison des première, deuxième, troisième et quatrième COI ; et
utiliser l'une des première, deuxième, troisième ou quatrième composantes de bande passante sélectionnée pour filtrer les signaux HS supplémentaires.

7. IMD selon l'une quelconque des revendications 1 à 6, dans lequel les un ou plusieurs processeurs (220) sont en outre configurés pour traiter une affection physiologique sur la base des signaux HS supplémentaires.

8. IMD selon l'une quelconque des revendications 1 à 7, dans lequel la première COI est une COI sélectionnée dans le groupe constitué par l'amplitude S1, l'amplitude S2, l'amplitude S3, l'intervalle S1 à S2, l'intervalle S2 à S3 et l'intervalle S3 à S1, et la deuxième COI est la COI sélectionnée.

9. IMD selon l'une quelconque des revendications 1 à 8, dans lequel l'axe est un premier axe et le capteur HS (270) comprend un deuxième axe et un troisième axe, et les un ou plusieurs processeurs (220) sont en outre configurés pour :
faire fonctionner l'IMD (100) dans un mode veille dans lequel aucun signal HS n'est détecté ;
faire fonctionner l'IMD (100) dans un mode mono-axe, dans lequel des signaux HS sont uniquement obtenus à partir du premier axe ; et
fonctionner dans un mode à trois axes, dans lequel des signaux HS sont obtenus séquentiellement à partir du premier axe puis à partir du deuxième axe.

10. IMD selon l'une quelconque des revendications 1 à 9, dans lequel les un ou plusieurs processeurs (220) sont en outre configurés pour :
obtenir des données HS provenant des signaux HS supplémentaires ; et
communiquer les données HS à un premier dispositif externe (1004).

11. IMD selon la revendication 10, dans lequel pour obtenir les données HS provenant des signaux HS supplémentaires, les un ou plusieurs processeurs (220) sont en outre configurés pour :
déterminer qu'un patient dort sur la base de mesures d'activité unidimensionnelle ou de tendance de posture en comparant des données d'accéléromètre liées au mouvement ou à la posture du patient à des données d'accéléromètre obtenues précédemment représentatives d'un patient endormi ; et
enregistrer les signaux HS supplémentaires pendant une période déterminée en réponse à la détermination du fait que le patient dort.

12. IMD selon l'une quelconque des revendications 1 à 11, dans lequel pour sélectionner l'une des première et deuxième bandes passantes, les un ou plusieurs processeurs (220) sont configurés pour déterminer un premier signal HS de moyenne d'ensemble lié à la première composante HS de bande passante et déterminer un second signal HS de moyenne d'ensemble lié à la deuxième composante HS de bande passante et comparer le premier signal HS de moyenne d'ensemble et le second signal HS de moyenne d'ensemble.

13. IMD selon l'une quelconque des revendications 1 à 12, dans lequel les un ou plusieurs processeurs (220) sont en outre configurés pour :
déterminer un premier temps d'activation électromécanique, EMAT, sur la base de la première COI du battement de cœur ;
déterminer un deuxième EMAT sur la base de la deuxième COI du battement de cœur ;
sélectionner l'un du premier EMAT ou du deuxième EMAT pour fournir un EMAT sélectionné sur la base d'une caractéristique partagée du premier EMAT et du deuxième EMAT ; et
obtenir les signaux HS supplémentaires pendant la seconde période de temps sur la base de l'EMAT sélectionné.

14. IMD selon l'une quelconque des revendications 1 à 12, dans lequel les un ou plusieurs processeurs (220) sont en outre configurés pour :
déterminer un premier temps d'activation électromécanique, EMAT, sur la base de la première composante HS de bande passante ;
déterminer un deuxième EMAT sur la base de la deuxième composante HS de bande passante ;
déterminer un troisième EMAT sur la base d'une troisième composante HS de bande passante ;
sélectionner l'un du premier EMAT, du deuxième EMAT ou du troisième EMAT pour fournir un EMAT sélectionné sur la base d'une caractéristique partagée du premier EMAT, du deuxième EMAT et du troisième EMAT ; et
obtenir les signaux HS supplémentaires sur la base de l'EMAT sélectionné.

15. IMD selon l'une quelconque des revendications 1 à 12, dans lequel les un ou plusieurs processeurs (220) sont en outre configurés pour :
déterminer un premier temps d'activation électromécanique, EMAT, sur la base de la première composante HS de bande passante et de la première COI du battement de cœur ;
déterminer au moins l'un parmi 1) un deuxième EMAT sur la base de la deuxième composante HS de bande passante et de la deuxième COI du battement de cœur ou 2) un troisième EMAT sur la base d'une troisième composante HS de bande passante et d'une troisième COI du battement de cœur ;
sélectionner l'un du premier EMAT, du deuxième EMAT ou du troisième EMAT pour fournir un EMAT sélectionné sur la base d'une caractéristique partagée du premier EMAT, du deuxième EMAT et du troisième EMAT ; et
obtenir les signaux HS supplémentaires sur la base de l'EMAT sélectionné.
